# EUROPEAN PATENT APPLICATION

(11) **EP 4 574 840 A1**
(43) Date of publication of application: **25.06.2025**
(21) Application number: 23854873.9
(22) Date of filing: 14.08.2023
(51) Int. Cl.: C07K 16/00, C07K 16/46, C12N 1/15, C12N 15/13, C12N 15/62, C12N 15/63, C12N 15/80, C12P 21/08

(54) **METHOD FOR PRODUCING IMMUNOGLOBULIN OR MULTIMER IMMUNOGLOBULIN USING FILAMENTOUS FUNGUS**

(30) Priority: 15.08.2022 JP 2022129448
(71) Applicant: The University of Tokyo, Bunkyo-ku, Tokyo 113-8654 (JP)
(72) Inventor: SHINKURA Reiko, Tokyo 113-8654 (JP); MARUYAMA Jun-ichi, Tokyo 113-8654 (JP); YU Lingxiao, Tokyo 113-8654 (JP)
(74) Representative: Grünecker Patent- und Rechtsanwälte PartG mbB
(86) International application number: PCT/JP2023/029403
(87) International publication number: WO 2024/038839

(57) **Abstract**

The present disclosure relates to a method for producing an immunoglobulin using a filamentous fungus, a filamentous fungus that can be used in the production method, and a method for producing the filamentous fungus. More specifically, the present disclosure relates to a method for producing an immunoglobulin using a filamentous fungus containing a nucleic acid fragment encoding an immunoglobulin in a genome, a filamentous fungus containing a nucleic acid fragment encoding a mammalian immunoglobulin in a genome and producing a mammalian immunoglobulin, and a method for producing a modified filamentous fungus, the method including inserting a nucleic acid sequence (typically two or more copies) encoding the immunoglobulin into a genome of a filamentous fungus to be modified.

## Description

### Technical Field

The present disclosure relates to a method for producing an immunoglobulin or a multimeric immunoglobulin using a filamentous fungus, a filamentous fungus that can be used in the production method, and a method for producing the filamentous fungus.

### Background Art

So far, transgenic animals, mammalian cell cultures, plants, or the like have been used for the production of beneficial proteins such as antibodies. However, transgenic animals and mammalian cell cultures have a risk of being contaminated with viruses, prions, and the like. In addition, it is difficult to expand the scale in a production system using the animals, animal cells, or plants, and in a genetically modified plant, it takes about 10 months to produce a recombinant protein, while in a mammalian cell culture, it takes about 3 months.

A koji mold (Aspergillus oryzae), which is a type of filamentous fungus, is a filamentous fungus that has been used from long ago in the production of traditional brewing products in Japan such as Japanese alcohol, soy sauce, and miso, and is a microorganism certified as a national fungus by the Brewing Society of Japan (Non Patent Literature 1: Kita moto, 2002). A. oryzae has excellent protein secretion ability, and useful proteins are actually produced using A. oryzae (Non Patent Literature 2: Ito et al., 2007, Non Patent Literature 3: Nakajima et al., 2006, Non Patent Literature 4: Tsuchiya et al., 1992, and Non Patent Literature 5: Tsuchiya et al., 1994). In addition, A. oryzae is recognized as a highly safe microorganism by the World Health Organization (WHO) and the Food and Agriculture Organization (FAO) (Non Patent Literature 6: Barbesgaard et al., 1992), and is also used for production of enzyme agents and metabolic compounds for foods, chemical products, and medical products.

Heterologous proteins produced by filamentous fungi include a wide range of proteins such as crystalline cellulose mitigating proteins derived from heterologous filamentous fungi (Non Patent Literature 7: Chen et al. (2010) Appl Environ Microbiol, Patent Literature 1: JP 2009-207368 A), taste-modifying proteins derived from plants (Non Patent Literature 3: Nakajima et al. (2006) Appl Environ Microbiol and Patent Literature 2: WO 2005/073372 A), and human lysozyme proteins (Patent Literature 3: WO 2007/099776 A). It has also been attempted to produce antibody molecules (immunoglobulins) such as IgG using Aspergillus niger and A. oryzae, which are filamentous fungi (Patent Literature 4, WO 2003/089614 A and Non Patent Literature 8: Huynh et al. (2020), Fungal Biol. Biotechnol).

The immunoglobulin is classified into five types of IgG, IgA, IgM, IgD, and IgE depending on a difference in structure of a heavy chain constant region, and each type has different chemical properties. In particular, it is known that IgM and IgA immunoglobulins function in a monomer state or a multimer state in a living body. The globulin in the multimer state is called a multimeric immunoglobulin.

### Citation List

### Patent Literature

Patent Literature 1: JP 2009-207368 A
Patent Literature 2: WO 2005/073372 A
Patent Literature 3: WO 2007/099776 A
Patent Literature 4: WO 2003/089614 A

### Non Patent Literature

Non Patent Literature 1: Kitamoto, Kitamoto, Adv Appl Microbiol (2002), 51:129-153
Non Patent Literature 2: Ito et al., 2007. Biochem Biophys Res Commun (2007), 360:407-411
Non Patent Literature 3: Nakajima et al, Appl Environ Microbiol (2006), 72:3716-3723
Non Patent Literature 4: Tsuchiya et al., Appl Microbiol Biotechnol (1992), 38:109-114
Non Patent Literature 5: Tsuchiya et al., Biosci Biotechnol Biochem (1994), 58:895-899
Non Patent Literature 6: Barbesgaard et al., Appl Microbiol Biotechnol (1992), 36:569-572
Non Patent Literature 7: Chen et al., Appl Environ Microbiol (2010), 76(8):2556-2561.
Non Patent Literature 8: Huynh et al. (2020) Fungal Biol. Biotechnol. (2020) 7:7 https://doi.org/10.1186/s40694-020-00098-w

### Summary of Invention

### Technical Problem

The present disclosure provides a method for producing an immunoglobulin or a multimeric immunoglobulin using a filamentous fungus. In another aspect, the present disclosure provides a filamentous fungus that can be used in the production method or a method for producing the filamentous fungus.

### Solution to Problem

As a result of intensive studies by the inventors of the present disclosure, it has been unexpectedly found that, by culturing a filamentous fungus containing a nucleic acid sequence encoding an immunoglobulin on a genome, an immunoglobulin or multimeric immunoglobulin retaining a physiological activity, for example, a bacterial growth inhibitory activity or the like can be produced, the immunoglobulin or the multimeric immunoglobulin can be secreted into a cell culture medium, and the produced immunoglobulin or multimeric immunoglobulin can be separated to utilize the physiological activity.

Based on this finding, in one aspect, the present disclosure provides a method for producing a multimeric immunoglobulin using a filamentous fungus containing a nucleic acid fragment encoding an immunoglobulin in a genome.

In addition, in another aspect, the present disclosure provides a filamentous fungus containing a nucleic acid fragment encoding a mammalian immunoglobulin in a genome and producing a multimeric mammalian immunoglobulin.

In addition, in still another aspect, the present disclosure provides a method for producing a filamentous fungus encoding an immunoglobulin in a genome, the method including inserting a nucleic acid sequence encoding the immunoglobulin into a genome of a filamentous fungus to be modified.

In addition, in still another aspect, the present disclosure provides a filamentous fungus containing a nucleic acid fragment encoding an immunoglobulin in a genome and producing an immunoglobulin.

More specifically, the present disclosure provides the following.

### [Item 1]

A method for producing a multimeric immunoglobulin, the method including:
a step of preparing a filamentous fungus containing a nucleic acid fragment encoding an immunoglobulin in a genome;
a step of culturing the filamentous fungus; and
a step of separating a multimeric immunoglobulin in a culture medium from other culture medium components.

### [Item 2]

The method according to Item 1, in which the filamentous fungus contains two or more copies of the nucleic acid fragment encoding the immunoglobulin in the genome.

### [Item 3]

The method according to Item 1 or 2, in which the two or more copies of the nucleic acid fragment encoding the immunoglobulin are inserted into a single region of the genome of the filamentous fungus.

### [Item 4]

The method according to Item 1, in which the immunoglobulin is IgA or IgM having a bacterial growth inhibitory action.

### [Item 5]

The method according to Item 1, in which in the immunoglobulin, a heavy chain lacks a partial or entire region of a C-terminal tail piece.

### [Item 6]

A filamentous fungus containing a nucleic acid fragment encoding an immunoglobulin in a genome, which is used in the method according to Item 1 or 2.

### [Item 7]

A multimeric immunoglobulin or a filamentous fungus containing a multimeric immunoglobulin, the multimeric immunoglobulin being produced by the method according to Item 1 or 2.

### [Item 8]

A composition containing a multimeric immunoglobulin or a filamentous fungus containing a multimeric immunoglobulin, the multimeric immunoglobulin being produced by the method according to Item 1 or 2.

### [Item 9]

A method for producing a filamentous fungus containing a nucleic acid fragment encoding an immunoglobulin in a genome, the method including:
a step of preparing a filamentous fungus to be modified;
a step of inserting two or more copies of the nucleic acid fragment encoding the immunoglobulin in the genome into the filamentous fungus to be modified by a single-stage genome modification manipulation; and
a step of confirming that the genetically modified filamentous fungus into which the nucleic acid fragment is inserted secretes a multimeric immunoglobulin into a culture medium.

### [Item 10]

A genetically modified filamentous fungus containing two or more copies of a nucleic acid fragment encoding an immunoglobulin in a genome, in which the filamentous fungus is capable of producing a multimeric immunoglobulin.

### [Item 11]

The filamentous fungus according to Item 10, in which the immunoglobulin is IgA or IgM.

### [Item 12]

A composition containing the filamentous fungus according to Item 10 or 11.

### [Item 13]

A composition containing a culture supernatant of the filamentous fungus according to Item 10 or 11.

### Advantageous Effects of Invention

The present disclosure has an effect of providing a method for producing an immunoglobulin or a multimeric immunoglobulin using a filamentous fungus. In another aspect, the present disclosure has an effect of providing a filamentous fungus that can be used in the production method or a method for producing the filamentous fungus.

### Brief Description of Drawings

Fig. 1 is a schematic diagram illustrating a configuration of a donor DNA plasmid pwAup-DN H+L+J(co) for producing a W27 IgA antibody into a dimer.
Fig. 2 is a schematic diagram illustrating a configuration of a genome editing plasmid pRGE-gwAup for introducing an expression cassette of a W27 IgA antibody.
Fig. 3 is a diagram illustrating the results of Western analysis of a culture supernatant of a dimeric IgA antibody-producing strain and a concentrated sample thereof.
Fig. 4 is a diagram illustrating a nucleic acid sequence of W27 IgA H of a donor DNA plasmid pwAup-DN H+L+J(co).
Fig. 5 is a diagram illustrating an amino acid sequence of W27 IgA H of a donor DNA plasmid pwAup-DN H+L+J(co).
Fig. 6 is a diagram illustrating a nucleic acid sequence of W27 IgA L of a donor DNA plasmid pwAup-DN H+L+J(co).
Fig. 7 is a diagram illustrating an amino acid sequence of W27 IgA L of a donor DNA plasmid pwAup-DN H+L+J(co).
Fig. 8 is a diagram illustrating a nucleic acid sequence of W27 IgA J of a donor DNA plasmid pwAup-DN H+L+J(co).
Fig. 9 is a diagram illustrating an amino acid sequence of W27 IgA J of a donor DNA plasmid pwAup-DN H+L+J(co).
Fig. 10 is a schematic diagram illustrating a configuration of a donor DNA plasmid pwAup-DN HAAComp+L(co) for producing a W27 IgA antibody into a pentamer.
Fig. 11 is a diagram illustrating the results of Western analysis of a culture supernatant of a pentamer IgA antibody-producing strain and a concentrated sample thereof.
Fig. 12 is a diagram illustrating a nucleic acid sequence of W27 IgA H of a donor DNA plasmid pwAup-DN HAAComp+L(co) .
Fig. 13 is a diagram illustrating an amino acid sequence of W27 IgA H of a donor DNA plasmid pwAup-DN HAAComp+L(co) .
Fig. 14 is a diagram illustrating a nucleic acid sequence of W27 IgA L of a donor DNA plasmid pwAup-DN HAAComp+L(co) .
Fig. 15 is a diagram illustrating an amino acid sequence of W27 IgA L of a donor DNA plasmid pwAup-DN HAAComp+L(co) .
Fig. 16 is a diagram illustrating the results of studying an influence of a configuration of an H chain C-terminal region of IgA and a configuration of a linker on a multimer formation.
Fig. 17 is a diagram illustrating the results of SDS-PAGE of a purified multimeric W27 IgA secreted from a dimer-expression-modified koji mold and a dimer-expression-modified CHO cell in a reduced state (left) and a non-reduced state (right), and CBB staining.
Fig. 18 is a diagram illustrating the results of SDS-PAGE of a purified multimeric W27 IgA secreted from a pentamer-expression-modified koji mold and a pentamer-expression-modified CHO cell in a non-reduced state, and CBB staining.
Fig. 19 is a diagram illustrating a binding force of a multimeric W27 IgA secreted from a dimer-expression-modified koji mold and a dimer-expression-modified CHO cell to E. coli.
Fig. 20 is a diagram illustrating a binding force of multimeric W27 IgA secreted from a pentamer-expression-modified koji mold, a pentamer-expression-modified CHO cell, a dimer-expression-modified koji mold, and a dimer-expression-modified CHO cell to E. coli.
Fig. 21 is a diagram illustrating an E. coli growth inhibitory effect of a multimeric W27 IgA secreted from a dimer-expression-modified koji mold and a dimer-expression-modified CHO cell.
Fig. 22 is a diagram illustrating a C. difficile growth inhibitory effect of a multimeric W27 IgA secreted from a dimer-expression-modified koji mold and a dimer-expression-modified CHO cell.

### Description of Embodiments

The inventors of the present disclosure have attempted to produce a multimeric immunoglobulin using a filamentous fungus based on the existing knowledge, but unlike a monomer immunoglobulin, it was not possible to produce a multimeric immunoglobulin, and particularly, it was not possible to produce a multimeric immunoglobulin by secreting a multimeric immunoglobulin having physiological activity into a cell culture medium and isolating the multimeric immunoglobulin. As a result of their own trial and error, the inventors of the present disclosure have surprisingly found that, by culturing a filamentous fungus containing a nucleic acid sequence encoding an immunoglobulin on a genome, a multimeric immunoglobulin having a physiological activity is secreted into a cell culture medium, and the multimeric immunoglobulin can be separated to utilize the physiological activity. Typically, a filamentous fungus containing a nucleic acid sequence encoding an immunoglobulin on a genome contains two or more copies of a nucleic acid sequence encoding an immunoglobulin on a genome.

### (Definition)

In the present specification, in a case where a plurality of ranges of numerical values are indicated, a range including any combination of a lower limit value and an upper limit value of the plurality of ranges is also meant in the same manner.

Unless defined otherwise, all technical terms and scientific terms used in the present specification have the same meanings as commonly understood by those skilled in the technical field to which the present invention pertains.

In the present specification, a case where the term "about" used in connection with a numerical value x means that the value may vary, for example, within 10%, 9%, 8%, 7%, 6%, 5%, 4%, 3%, 2%, 1%, 0.5%, 0.1%, 0.05%, or 0.01%.

In the present specification, the term "containing" has the same meaning as commonly understood by those skilled in the technical field to which the present invention pertains, but includes, for example, "containing" and "consisting of", and specifically means that a composition "containing" A may also contain B as another component, in addition to containing only A.

In the present specification, the "separating" has the same meaning as commonly understood by those skilled in the technical field to which the present invention belongs, and means, for example, selectively obtaining certain components from a mixed composition and excluding other components. In the present specification, in a case where an immunoglobulin is separated from a culture medium, "separating" may have the same meaning as "isolating", "purifying", "enriching", and "concentrating".

In the present specification, the "protein" refers to a substance composed of a polypeptide in which amino acids are linked by peptide bonds. In the present specification, a molecular weight of the protein is not particularly limited. Thus, in the present specification, the term "protein" also includes the meaning of a polypeptide and a peptide.

### (Method for Producing Immunoglobulin)

In one aspect, the present disclosure provides a method for producing an immunoglobulin using a filamentous fungus containing a nucleic acid fragment encoding an immunoglobulin in a genome.

The method for producing an immunoglobulin of the present disclosure includes:
a step of preparing a filamentous fungus containing a nucleic acid fragment encoding an immunoglobulin in a genome;
a step of culturing the filamentous fungus; and
a step of separating an immunoglobulin in a culture medium from other culture medium components.

As the filamentous fungus encoding the immunoglobulin in the genome used in the method for producing an immunoglobulin of the present disclosure, a filamentous fungus produced by a method for producing a modified filamentous fungus for producing a multimeric mammalian immunoglobulin described below can be typically used. The type of immunoglobulin produced by the method of the present disclosure is not particularly limited, and can be selected from, for example, IgG, IgA, IgM, IgD, or IgE. In a preferred aspect, the immunoglobulin is IgA or IgM. In a preferred aspect, the resulting immunoglobulin has a physiological activity, for example, a binding activity to an antigen. In a preferred aspect, the immunoglobulin is IgA or IgM having a physiological activity, for example, a binding activity to an antigen.

In the step of culturing the filamentous fungus of the method for producing an immunoglobulin of the present disclosure, conditions for culturing the filamentous fungus, for example, a composition of a medium, a culture temperature, a medium pH, and shaking conditions can be arbitrarily adjusted and optimized based on technologies known to those skilled in the art. By optimizing these conditions, the amount, yield, quality, and the like of the immunoglobulin can be improved, and an immunoglobulin having desired properties in stability and the like can be produced.

In one aspect, a pH of the medium in which the filamentous fungus is cultured can be adjusted at any pH of 4 to 11. Preferably, the pH of the medium is selected from a range of 4.0 to 11.0, 4.5 to 10.0, 5.0 to 9.0, 5.5 to 8.5, or 6.0 to 8.0. In one aspect, the pH of the medium can be set within a range of 5.0 or more, 5.5 or more, 6.0 or more, 6.5 or more, 7.0 or more, 7.5 or more, 8.0 or more, 8.5 or more, 9.0 or more, or 9.5 or more, and 11.0 or less, 10.5 or less, 10.0 or less, 9.5 or less, 9.0 or less, 8.5 or less, 8.0 or less, 7.5 or less, 7.0 or less, 6.5 or less, 6.0 or less, 5.5 or less, or 5.0 or less, or any combination of an upper limit and a lower limit thereof.

In the method for producing an immunoglobulin of the present disclosure, in the step of separating the immunoglobulin in the culture medium from the other culture medium components, the immunoglobulin produced by the bacterial cell body (inside the bacterial cell body or preferably outside the bacterial cell body) can be separated according to a conventional method. Since the filamentous fungus used in the method for producing an immunoglobulin of the present disclosure typically secretes and produces the immunoglobulin to the outside of the bacterial cell body, it is not essential to perform a treatment of disrupting the bacterial cell body for isolation and purification of the immunoglobulin. In a preferred aspect, the method for producing an immunoglobulin of the present disclosure does not include a treatment for disrupting the bacterial cell body. The filamentous fungus used in the method for producing an immunoglobulin of the present disclosure may produce an immunoglobulin in a bacterial cell body. A filamentous fungus containing an immunoglobulin in a bacterial cell body is suitable for oral administration. When the filamentous fungus containing an immunoglobulin in a bacterial cell body is orally administered, the bacterial cell body may be disrupted before administration, or may not be disrupted.

The manipulation of separating the immunoglobulin from the culture medium can be performed using various methods commonly utilized in the technical field of the present disclosure to separate proteins. For example, techniques such as affinity chromatography, ion exchange chromatography, hydrophobic interaction chromatography, ethanol precipitation, reverse phase HPLC, chromatography on silica or with a cation exchange resin such as DEAE, chromatofocusing, SDS-PAGE, ammonium sulfate precipitation, and gel filtration can be used alone or in combination.

The immunoglobulin produced by the method of the present disclosure may be immunoglobulins of various organisms. For example, the immunoglobulin may be an immunoglobulin of a human, a non-human primate, a mouse, a rat, a guinea pig, a rabbit, a hamster, a dog, a cat, a weasel, a cow, a pig, a horse, a deer, a wild boar, a sheep, a goat, a camel, or the like. In a preferred aspect, the immunoglobulin is a human immunoglobulin.

The immunoglobulin produced by the method of the present disclosure may contain a natural amino acid sequence in a heavy chain or a light chain, or may contain a mutation with respect to the natural amino acid sequence. The mutation includes addition, deletion, and substitution of an amino acid, and includes shortening of an N-terminus or C-terminus. The properties such as antigen binding ability and stability of the immunoglobulin to be produced can be adjusted by the mutation.

### (Method for Producing Multimeric Immunoglobulin)

In one aspect, the present disclosure provides a method for producing a multimeric immunoglobulin using a filamentous fungus containing a nucleic acid fragment encoding an immunoglobulin in a genome.

The method for producing a multimeric immunoglobulin of the present disclosure includes:
a step of preparing a filamentous fungus containing a nucleic acid fragment encoding an immunoglobulin in a genome;
a step of culturing the filamentous fungus; and
a step of separating a multimeric immunoglobulin in a culture medium from other culture medium components.

As the filamentous fungus encoding the immunoglobulin in the genome used in the method for producing a multimeric immunoglobulin of the present disclosure, a filamentous fungus produced by a method for producing a modified filamentous fungus for producing an immunoglobulin described below can be typically used.

In the step of culturing the filamentous fungus of the method for producing a multimeric immunoglobulin of the present disclosure, conditions for culturing the filamentous fungus, for example, a composition of a medium, a culture temperature, a medium pH, and shaking conditions can be arbitrarily adjusted and optimized based on techniques known to those skilled in the art. By optimizing these conditions, the amount, yield, quality, and the like of the immunoglobulin can be improved, and an immunoglobulin having desired properties in stability and the like can be produced.

In one aspect, a pH of the medium in which the filamentous fungus is cultured can be adjusted at any pH of 4 to 11.

Preferably, the pH of the medium can be selected from a range of 4.0 to 11.0, 4.5 to 10.0, 5.0 to 9.0, 5.5 to 8.5, or 6.0 to 8.0. In one aspect, the pH of the medium can be set within a range of 5.0 or more, 5.5 or more, 6.0 or more, 6.5 or more, 7.0 or more, 7.5 or more, 8.0 or more, 8.5 or more, 9.0 or more, or 9.5 or more, and 11.0 or less, 10.5 or less, 10.0 or less, 9.5 or less, 9.0 or less, 8.5 or less, 8.0 or less, 7.5 or less, 7.0 or less, 6.5 or less, 6.0 or less, 5.5 or less, or 5.0 or less, or any combination of an upper limit and a lower limit thereof.

In the method for producing a multimeric immunoglobulin of the present disclosure, in the step of separating the multimeric immunoglobulin in the culture medium from the other culture medium components, the multimeric immunoglobulin secreted and produced outside the bacterial cell body can be separated according to a conventional method. Since the filamentous fungus used in the method for producing a multimeric immunoglobulin of the present disclosure typically secretes and produces the multimeric immunoglobulin to the outside of the bacterial cell body, it is not essential to perform a treatment of disrupting the bacterial cell body. In a preferred aspect, the method for producing a multimeric immunoglobulin of the present disclosure does not include a treatment for disrupting the bacterial cell body.

The manipulation of separating the multimeric immunoglobulin from the culture medium can be performed using various methods commonly utilized in the technical field of the present disclosure to separate proteins. For example, techniques such as affinity chromatography, ion exchange chromatography, hydrophobic interaction chromatography, ethanol precipitation, reverse phase HPLC, chromatography on silica or with a cation exchange resin such as DEAE, chromatofocusing, SDS-PAGE, ammonium sulfate precipitation, and gel filtration can be used alone or in combination.

The separated immunoglobulin in a multimer form can be confirmed using various methods commonly used in the technical field of the present disclosure. For example, it can be confirmed that the immunoglobulin is a multimer by analyzing the immunoglobulin separated from the culture medium by sodium dodecyl sulfate-polyacrylamide gel electrophoresis (SDS-PAGE) in a non-reduced state.

The multimeric immunoglobulin produced by the method of the present disclosure may be immunoglobulins of various organisms. For example, the immunoglobulin may be an immunoglobulin of a human, a non-human primate, a mouse, a rat, a guinea pig, a rabbit, a hamster, a dog, a cat, a weasel, a cow, a pig, a horse, a deer, a wild boar, a sheep, a goat, a camel, or the like. The multimeric immunoglobulin is preferably IgA or IgM.

The multimeric immunoglobulin produced by the method of the present disclosure may contain a natural amino acid sequence in a heavy chain or a light chain, or may contain a mutation with respect to the natural amino acid sequence. The mutation includes addition, deletion, and substitution of an amino acid, and includes shortening of an N-terminus or C-terminus. In one aspect, a heavy chain of an immunoglobulin lacking a partial or entire C-terminal tail piece can be used. The properties such as multimer forming ability, antigen binding ability, and stability of the multimeric immunoglobulin to be produced can be adjusted by the mutation.

The multimeric immunoglobulin produced by the method of the present disclosure may contain a heterologous amino acid sequence to promote multimerization. As such a heterologous amino acid sequence, for example, an amino acid sequence forming a coiled-coil domain can be used. In one aspect, the heterologous amino acid sequence can be derived from any of cartilage oligomer substrate protein (COMP), mannose-binding protein A, coiled-coil serine-rich protein 1, polypeptide release factor 2, SNAP-25, SNARE, Lac repressor, or apolipoprotein E. In a preferred aspect, as the heterologous amino acid sequence for promoting multimerization, for example, a coiled-coil assembly domain of cartilage oligomer substrate protein (COMP) described in JP 2016-512309 A can be used. The heterologous amino acid sequence can be fused to an N-terminus or a C-terminus, preferably a C-terminus, of an immunoglobulin protein. Various linker sequences can be used for the fusion as long as a desired physiological function of the multimeric immunoglobulin is not lost, and may not be used. In one aspect, a linker consisting of an amino acid sequence SSADDAKKDAAKKDDAKKDDAKKDAS or a linker consisting of an amino acid sequence SSADDAAADAAAADDAAADDAAADAS can be used. In one aspect, the heterologous sequence linker or linker for promoting multimerization can be fused to a shortened N-terminus or C-terminus of the immunoglobulin.

### (Modified Filamentous Fungus Producing Immunoglobulin or Multimeric Immunoglobulin)

In another aspect, the present disclosure provides a modified filamentous fungus containing a nucleic acid fragment encoding a mammalian immunoglobulin in a genome and producing an immunoglobulin or a multimeric immunoglobulin. The modified filamentous fungus can be used in (Method for Producing Immunoglobulin) or (Method for Producing Multimeric Immunoglobulin) of the present disclosure, and produces or secretes an immunoglobulin or a multimeric immunoglobulin inside the bacterial cell body or outside the bacterial cell body.

The modified filamentous fungus producing a multimeric immunoglobulin of the present disclosure typically contains two or more copies of a nucleic acid fragment encoding an immunoglobulin on a genome. In one aspect, the modified filamentous fungus of the present disclosure contains 2 or more copies, 3 or more copies, 4 or more copies, 5 or more copies, 6 or more copies, 7 or more copies, 8 or more copies, 9 or more copies, or 10 or more copies of a nucleic acid sequence encoding an immunoglobulin on a genome. The higher the copy number of the nucleic acid sequence encoding the immunoglobulin contained on the genome, the easier it is to obtain a modified filamentous fungus that produces or secretes a multimeric immunoglobulin more efficiently. A filamentous fungus producing a dimeric immunoglobulin contains 2 or more copies, 3 or more copies, 4 or more copies, 5 or more copies, 6 or more copies, 7 or more copies, 8 or more copies, 9 or more copies, or 10 or more copies of a nucleic acid sequence encoding an immunoglobulin on a genome. A filamentous fungus producing a trimer immunoglobulin contains 3 or more copies, 4 or more copies, 5 or more copies, 6 or more copies, 7 or more copies, 8 or more copies, 9 or more copies, or 10 or more copies of a nucleic acid sequence encoding an immunoglobulin on a genome. A filamentous fungus producing a pentamer immunoglobulin contains 5 or more copies, 6 or more copies, 7 or more copies, 8 or more copies, 9 or more copies, or 10 or more copies of a nucleic acid sequence encoding an immunoglobulin on a genome. A nucleic acid fragment encoding an immunoglobulin is inserted or substituted into a single region or a plurality of regions on a genome. Typically, a nucleic acid fragment encoding an immunoglobulin is inserted or substituted into a single region on a genome. The single region on the genome means one continuous region on the genome of the filamentous fungus. In one aspect, the single region on the genome has, for example, a size on the order of one locus. For example, the single region on the genome has a size of about 3 to 12 kbp, but is not particularly limited.

The modified filamentous fungus producing an immunoglobulin or a multimeric immunoglobulin of the present disclosure can be obtained by culturing a filamentous fungus subjected to a genome modification treatment and identifying a filamentous fungus that produces or secretes an immunoglobulin or a multimeric immunoglobulin in a bacterial cell body or in a culture medium.

The multimer form of the multimeric immunoglobulin secreted by the filamentous fungus subjected to the genome modification treatment can be confirmed using various methods generally used in the technical field of the present disclosure. For example, it can be confirmed that the multimeric immunoglobulin is a multimer by analyzing the immunoglobulin separated from the culture medium by sodium dodecyl sulfate-polyacrylamide gel electrophoresis (SDS-PAGE) in a non-reduced state.

For the genome modification technology (genome editing technology) used for producing the genetically modified filamentous fungus of the present disclosure, various methods commonly used in the technical field of the present disclosure can be used. For example, CRISPR-Cas system, meganucleases (MNs), zinc finger nucleases (ZFN), transcription activation-like effector nucleases (TALENs), and the like can be used. In one aspect, it is preferable to use a modification technology in which a plasmid for genome modification does not remain in the modified filamentous fungus. For example, it is possible to obtain a genome-modified filamentous fungus in which a plasmid for genome modification does not remain in the filamentous fungus after modification by using a genome editing plasmid for producing a multi-stage multi-mutant strain disclosed in JP 2018-191551 A for a single-stage mutation manipulation or a multi-stage mutation manipulation.

The genome-modified filamentous fungus according to the present disclosure can be produced by modifying a genome of a conventionally known filamentous fungus. The filamentous fungus to be modified is not particularly limited. Non-limiting examples of the filamentous fungi that can be used include, but are not limited to, Aspergillus filamentous fungi such as Aspergillus aculeatus, Aspergillus luchuensis, Aspergillus caesiellus, Aspergillus candidus, Aspergillus carneus, Aspergillus clavatus, Aspergillus deflectus, Aspergillus fischerianus, Aspergillus flavus, Aspergillus fumigatus, Aspergillus glaucus, Aspergillus nidulans, Aspergillus niger, Aspergillus ochraceus, Aspergillus oryzae, Aspergillus parasiticus, Aspergillus penicilloides, Aspergillus restrictus, Aspergillus sojae, Aspergillus sydowi, Aspergillus tamari, Aspergillus terreus, Aspergillus ustus, and Aspergillus versicolor, Trichoderma filamentous fungi such as Trichoderma aggressivum, Trichoderma asperellum, Trichoderma atroviride, Trichoderma aureoviride, Trichoderma austrokoningii, Trichoderma brevicompactum, Trichoderma candidum, Trichoderma caribbaeum var. aequatoriale, Trichoderma caribbaeum var. Caribbaeum, Trichoderma catoptron, Trichoderma cremeum, Trichoderma ceramicum, Trichoderma cerinum, Trichoderma chlorosporum, Trichoderma chromospermum, Trichoderma cinnamomeum, Trichoderma citrinoviride, Trichoderma crassum, Trichoderma cremeum, Trichoderma dingleyeae, Trichoderma dorotheae, Trichoderma effusum, Trichoderma erinaceum, Trichoderma estonicum, Trichoderma fertile, Trichoderma gelatinosus, Trichoderma ghanense, Trichoderma hamatum, Trichoderma harzianum, Trichoderma helicum, Trichoderma intricatum, Trichoderma konilangbra, Trichoderma koningii, Trichoderma koningiopsis, Trichoderma longibrachiatum, Trichoderma longipile, Trichoderma minutisporum, Trichoderma oblongisporum, Trichoderma ovalisporum, Trichoderma petersenii, Trichoderma phyllostahydis, Trichoderma piluliferum, Trichoderma pleuroticola, Trichoderma pleurotum, Trichoderma polysporum, Trichoderma pseudokoningii, Trichoderma pubescens, Trichoderma reesei, Trichoderma rogersonii, Trichoderma rossicum, Trichoderma saturnisporum, Trichoderma sinensis, Trichoderma sinuosum, Trichoderma sp. MA 3642, Trichoderma sp. PPRI 3559, Trichoderma spirale, Trichoderma stramineum, Trichoderma strigosum, Trichoderma stromaticum, Trichoderma surrotundum, Trichoderma taiwanense, Trichoderma thailandicum, Trichoderma thelephoricolum, Trichoderma theobromicola, Trichoderma tomentosum, Trichoderma velutinum, Trichoderma virens, Trichoderma viride, and Trichoderma viridescens, Rhizomucor filamentous fungi such as Rhizomucor pusillus and Rhizomucor miehei, Penicillium filamentous fungi such as Penicillium notatum and Penicillium chrysogenum, Rhizopus fungi such as Rhizopus oryzae, Acremonium cellulolyticus, Humicola grisea, and Thermoaseus aurantiacus. In particular, it is preferable to use an Aspergillus filamentous fungus as an object to be modified, and in particular, it is preferable to use Aspergillus oryzae as an object to be modified.

In addition, the filamentous fungus to be modified may be a wild type strain or a mutant strain into which various mutations are introduced. For example, a mutant strain in which a vacuolar acid protease gene is disrupted, which is described in WO 2007/099776 A, or a mutant strain in which a function of an autophagy-related gene is reduced, which is disclosed in JP 2012-179011 A, may be used.

In another aspect, as the modified filamentous fungus of the present disclosure, a filamentous fungus modified in addition to a modification in which a nucleic acid sequence encoding an immunoglobulin is introduced into a genome can be used. For example, modifications such as disruption of a vacuolar acid protease gene described in WO 2007/099776 A and functional reduction of an autophagy-related gene disclosed in JP 2012-179011 A can be added.

The immunoglobulin or the multimeric immunoglobulin encoded by the nucleic acid fragment contained in the genome of the modified filamentous fungus of the present disclosure may be immunoglobulins of various organisms. For example, the immunoglobulin may be an immunoglobulin of a human, a non-human primate, a mouse, a rat, a guinea pig, a rabbit, a hamster, a dog, a cat, a weasel, a cow, a pig, a horse, a deer, a wild boar, a sheep, a goat, a camel, or the like. The immunoglobulin or the multimeric immunoglobulin is preferably IgA or IgM.

### (Method for Producing Modified Filamentous Fungus Producing Immunoglobulin or Multimeric Immunoglobulin)

In one aspect, the present disclosure provides a method for producing a filamentous fungus encoding an immunoglobulin in a genome, the method including inserting a nucleic acid sequence encoding the immunoglobulin into a genome of a filamentous fungus to be modified to produce a modified filamentous fungus producing an immunoglobulin or a multimeric immunoglobulin.

The method for producing a modified filamentous fungus of the present disclosure typically includes:
a step of preparing a filamentous fungus to be modified;
a step of inserting a nucleic acid fragment encoding an immunoglobulin into a genome of the filamentous fungus to be modified by a single-stage genome modification manipulation; and
a step of confirming that the genetically modified filamentous fungus into which the nucleic acid fragment is inserted secretes an immunoglobulin or a multimeric immunoglobulin into a culture medium.

In the method for producing a modified filamentous fungus of the present disclosure, the single-stage genome modification manipulation means not including two or more manipulations of transducing a nucleic acid fragment encoding an immunoglobulin into a filamentous fungus. Typically, the single-stage genome modification manipulation includes a step of transducing a nucleic acid fragment encoding an immunoglobulin into a filamentous fungus and a step of identifying the transduced filamentous fungus using a negative marker or a positive marker.

The method for producing a modified filamentous fungus of the present disclosure may further involve an additional genome modification manipulation as long as the method includes a step of inserting a nucleic acid fragment encoding an immunoglobulin into a genome of a filamentous fungus to be modified by a single-stage genome modification manipulation. For example, in a case where a modified filamentous fungus containing two or more copies of a nucleic acid fragment encoding an immunoglobulin is produced, in addition to the step of inserting two or more copies of the nucleic acid fragment encoding an immunoglobulin into the genome of the filamentous fungus to be modified by the single-stage genome modification manipulation, a step of transducing one copy of the nucleic acid fragment encoding the immunoglobulin by a single-stage genome modification manipulation may be involved. In one aspect, any other genome modification manipulation that is beneficial for the production of the immunoglobulin may be involved.

In one aspect, in the method for producing a modified filamentous fungus of the present disclosure, the genetic constitution of the filamentous fungus can be modified by a method other than the genome modification. For example, a heterologous gene may be transduced with a plasmid.

In the method for producing a modified filamentous fungus of the present disclosure, in a case where an additional genome modification or another modification is included, a plurality of genome modifications or modification steps may be included in combination, and the step may be performed before or after or both, the step of inserting the nucleic acid fragment encoding an immunoglobulin into the genome of the filamentous fungus to be modified.

The filamentous fungus prepared in the method for producing a modified filamentous fungus of the present disclosure is not particularly limited. For example, various filamentous fungi mentioned as genome modification targets in (Modified Filamentous Fungus Producing Immunoglobulin or Multimeric Immunoglobulin) can be used.

The genome modification manipulation used in the method for producing a modified filamentous fungus of the present disclosure is not particularly limited. For example, various genome modification technologies mentioned in (Modified Filamentous Fungus Producing Immunoglobulin or Multimeric Immunoglobulin) can be used.

Various vectors or nucleic acid constructs commonly used in the technical field of the present disclosure can be used as vectors or nucleic acid constructs used in the genome modification manipulation included in the method for producing a modified filamentous fungus of the present disclosure. For example, a vector containing a selectable marker gene, a cloning site, and a control region (a promoter or a terminator) disclosed in JP 2012-179011 A can be used. In one aspect, in a case where the CRISPR-Cas system is used for the genome modification manipulation, a vector containing a DNA fragment AMA1 derived from A. nidulans which enables autonomous replication of a plasmid and a DNA fragment designed to highly express Aoace2 gene which significantly deteriorates growth due to high expression, which is disclosed in JP 2018-191551 A, can be suitably used.

The step of confirming that the genetically modified filamentous fungus into which the nucleic acid fragment is inserted secretes the multimeric immunoglobulin into the culture medium, which is included in the method for producing a modified filamentous fungus of the present disclosure, can be performed using various methods commonly used in the technical field of the present disclosure. For example, it can be confirmed that the immunoglobulin is a multimer by analyzing the immunoglobulin separated from the culture medium by sodium dodecyl sulfate-polyacrylamide gel electrophoresis (SDS-PAGE) in a non-reduced state.

### (Composition)

The immunoglobulin or multimeric immunoglobulin produced using the modified filamentous fungus of the present invention can be provided as a composition containing a purified immunoglobulin, or can be provided as a composition containing an immunoglobulin or a modified filamentous fungus itself expressing a multimeric immunoglobulin, a composition containing a part or the whole of a culture solution of a modified filamentous fungus expressing an immunoglobulin or a multimeric immunoglobulin, or a composition containing a part or the whole of the modified filamentous fungus and the culture solution. The composition of the present invention is preferably provided in the form of, but not limited to, a pharmaceutical composition, a food composition, a feed composition, and the like. In one aspect, the pharmaceutical composition, the food composition, and the feed composition as the composition of the present invention each include a material composition used to produce a final product.

### (Pharmaceutical Composition)

The pharmaceutical composition according to the present invention is used for preventing or treating a disease or disorder by utilizing an effect of an immunoglobulin or a multimeric immunoglobulin produced using a modified filamentous fungus.

In one aspect, the pharmaceutical composition according to the present invention is used for treating a disease associated with C. difficile.

The disease associated with C. difficile is not particularly limited, examples thereof include an inflammatory bowel disease, ulcerative colitis, Crohn's disease, allergy, asthma, obesity, autoimmune disease, and neonatal necrotizing enterocolitis, and among them, an inflammatory bowel disease is preferable.

Such a pharmaceutical composition according to the present invention only needs to contain an effective amount of the immunoglobulin or multimeric immunoglobulin produced using the modified filamentous fungus according to the present invention, and can be appropriately set, for example, so that a content ratio of the antibody according to the present invention in 100 wt% of the pharmaceutical composition is in a range of 0.001 to 99.99 wt%, in consideration of the type of a target disease, a dosage form, an administration method, a target to be administered, a degree of symptoms of an administration subject, a degree of effect exerted by administration, and the like.

The term "effective amount" as used in the present specification means an amount by which the multimeric immunoglobulin produced using the modified filamentous fungus according to the present invention exhibits a desired physiological effect in a living body.

The pharmaceutical composition according to the present invention may contain a pharmaceutically acceptable carrier or additive together with the immunoglobulin or multimeric immunoglobulin produced using the modified filamentous fungus according to the present invention. The pharmaceutically acceptable carrier or additive means any carrier, diluent, excipient, suspending agent, lubricant, adjuvant, medium, delivery system, emulsifier, disintegrant, absorbent, preservative, surfactant, colorant, flavoring, or sweetener, and a known pharmaceutically acceptable carrier or additive may be employed.

Examples of the individual to be administered include, but are not limited to, mammals such as a human, a mouse, a rat, a guinea pig, a rabbit, a hamster, a dog, a cat, a weasel, a cow, and a pig, and birds such as a chicken.

A dose and administration method of the pharmaceutical composition vary depending on the type, gender, species, age, general condition, severity of the disease, degree of desired effect, and the like of the intestinal disease that affects the individual to be administered. Usually, the dosage may be appropriately set in a range of 0.001 to 100 mg/kg/day.

In addition, the administration method is not particularly limited, but it is preferable to directly administer to the digestive tract, and examples of such an administration method include oral administration, nasal administration, transmucosal administration, and enteral administration.

Note that the enteral administration is not limited to administration via the anus, and includes, for example, administration via a tube or the like inserted into the digestive tract from the outside of the individual like a gastrostomy or the like. A position where the digestive tract is inserted is not limited to the intestine, and examples thereof include esophagus, stomach, small intestine (including duodenum, jejunum, ileum, and the like), and large intestine (including cecum, colon, rectum, and the like).

Note that in the administration of such a pharmaceutical composition according to the present invention, the above amount may be administered once a day, or may be administered in several divided doses. In addition, an administration interval may be every day, every other day, every week, every other week, every 2 or 3 weeks, every month, every other month, or every 2 or 3 months as long as the therapeutic effect on the disease is obtained.

### (Food Composition and Feed Composition)

The food composition or the feed composition according to the present invention contains the immunoglobulin or multimeric immunoglobulin produced using the modified filamentous fungus according to the present invention. The food composition according to the present invention includes, in addition to a general food, all forms of foods ingested as foods, such as a food for specified health uses including a food for conditional specified health uses, a nutritional supplementary food, a functional food, and a food for a patient. By using such a food composition or feed composition, the physiological activity of the immunoglobulin or multimeric immunoglobulin produced using the modified filamentous fungus according to the present invention can be utilized. The food composition can be provided as a food composition labeled as for intestinal regulation, intestinal environment improvement, intestinal environment optimization improvement, intestinal spoilage prevention, or the like.

A blending ratio of the immunoglobulin or multimeric immunoglobulin produced using the modified filamentous fungus in such a food composition or feed composition is not particularly limited, and may be appropriately adjusted according to the form, use, and the like of the food composition or feed composition. Usually, the blending ratio may be about 0.001 to 99 wt% with respect to the total amount of the composition.

The intake amount of the food composition or the feed composition according to the present invention is not particularly limited, and can be set according to an intended effect, an intended degree thereof, other various conditions, and the like. For example, the amount of the immunoglobulin or multimeric immunoglobulin according to the present invention may be usually about 0.001 to 100 mg/kg/day, and the immunoglobulin or multimeric immunoglobulin may be taken once to several times a day.

Specific examples of the form of the food composition described above include, but are not limited to, drinks such as soft drinks, carbonated drinks, nutritional drinks, fruit drinks, lactic acid drinks, and milk drinks; frozen desserts such as ice cream, ice sorbet, and shaved ice; confectioneries such as candies, candies, gum, chocolate, tablet candies, snacks, biscuits, jelly, jam, cream, and baked confectioneries; noodles such as buckwheat noodles, thick noodles made from wheat flour, starch noodles, Chinese-style noodles, and instant noodles; fish or livestock processed foods such as fish cakes, ham, and sausage; dairy products such as processed milk products and fermented milk products; oil and fat and oil and fat processed foods such as salad oil, tempura oil, margarine, mayonnaise, shortening, whipped cream, and dressing; seasonings such as sauces and tare sauce; and soups, stews, salads, side dishes, furikake, pickles, bread, and cereal. In addition, in the cases of foods for specified health uses, nutritional supplementary foods, functional foods, and the like, examples thereof include powders, granules, capsules, troches, tablets, and syrups.

The food composition of the feed composition according to the present invention can be provided as a composition containing a purified immunoglobulin, or can be provided as a composition containing an immunoglobulin or a modified filamentous fungus itself expressing a multimeric immunoglobulin, a composition containing a part or the whole of a culture solution of a modified filamentous fungus expressing an immunoglobulin or a multimeric immunoglobulin, or a composition containing a part or the whole of the modified filamentous fungus and the culture solution. In one aspect, the food composition or the feed composition of the present invention is provided as a composition produced through a process of culturing, preferably fermenting, a filamentous fungus. In this case, a culture or a brewed and fermented product may be used as a food composition as it is, or a product obtained by purifying a part of the brewed and fermented product may be used as a food composition. For example, a solid component including a filamentous fungus can be filtered from a culture or a brewed and fermented product to provide as a liquid composition. Such a composition can include the filamentous fungus itself, a part of the filamentous fungus, an extract of the filamentous fungus, a component serving as a medium for the filamentous fungus, or a mixture thereof. Non-limiting examples of such a food composition include mirin, sake, amazake, miso, soy sauce, salt koji, rice koji, soy sauce koji, barley koji, soybean koji, koji natto, sake lees fish koji: fish sauce base, ragi (Indonesia), ketchup (Indonesian miso, no tomatoes), soy sauce: Hishio Jiuniang, sweet fermented rice, mochi koji, maki koji, cheonggukjang, or katsuobushi, and other miscellaneous.

The feed composition of the present invention can be provided as a feed composition labeled as for intestinal regulation, intestinal environment improvement, intestinal environment optimization improvement, intestinal spoilage prevention, or the like for various animals.

The specific form of the feed composition described above is not particularly limited, and for example, as long as the effect exhibited by the feed composition of the present invention described above is not impaired, a feed composition may be prepared by mixing with a normal feed, or as necessary, by mixing a component that can be blended with a normal feed, or a feed composition itself may be used as a feed. For example, pomace used for production of various filamentous fungi fermented foods can be blended with a feed.

### Examples

Hereinafter, the present invention will be described in more detail with reference to Example, but these Examples are merely illustrative and do not limit the present disclosure.

### Methods and Materials

### 1. DNA Manipulation Method

E. coli DH5α was used for DNA manipulation. PrimeSTARHS DNA polymerase (TaKaRa) was used for PCR. In-Fusion HD cloning kit (TaKaRa) was used for plasmid production. Plasmids were extracted by alkaline SDS method. The nucleic acid sequence was analyzed by FASMAC Co., Ltd.

### 2. Production of Genome Editing Plasmid for Gene Introduction into wA Gene

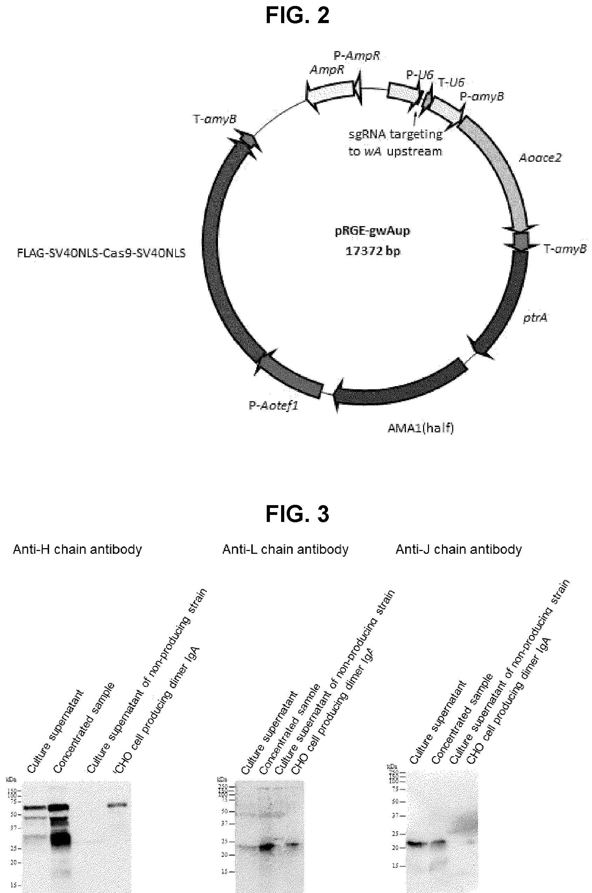

The genome editing plasmid pRGE-gwAup (Fig. 2) expressing sgRNA performing double-stranded DNA cleavage targeting the upstream of the wA gene was produced as described by Katayama et al. [Appl. Environ. Microbiol., 85:e01896-18, 2019]. A DNA fragment containing an sgRNA coding sequence and a U6 terminator was connected to ppAsATC9a2 cleaved with SmaI to produce pRGE-gRT6. A U6 promoter was connected to pRGE-gRT6 cleaved with SmaI to produce pRGE-gwAup.

### 3. Method for Producing Plasmid

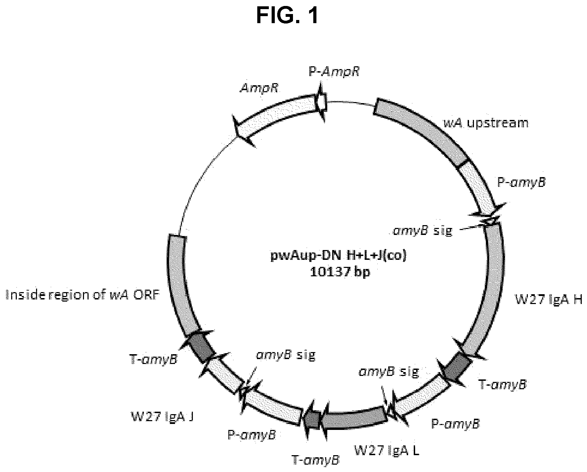

For transformation to obtain a dimeric IgA-producing strain, a donor DNA plasmid pwAup-DN H+L+J(co) (Fig. 1) was produced as follows.

First, plasmid pwAup-DN H+LlinkerSmaI(co) was constructed. A DNA fragment containing an amyB promoter, a signal sequence of AmyB, and a coding region of an H chain of W27 IgA was amplified. A DNA fragment containing an amyB terminator, an amyB promoter, a signal sequence of AmyB, a coding region of an L chain of W27 IgA, and an amyB terminator was amplified, and at the same time, a linker sequence and an SmaI cleavage site were also introduced into the 3' end of the amyB terminator. These two DNA fragments were connected by plasmid pwAup-DN cleaved with SmaI using In-Fusion HD cloning kit to produce pwAup-DN H+LlinkerSmaI(co).

Subsequently, plasmid pwAup-DN H+L+J(co) was constructed. A DNA fragment containing an amyB promoter, a signal sequence of AmyB, a coding region of a J chain of W27 IgA, and an amyB terminator was amplified. The DNA was connected to plasmid pwAup-DN H+LlinkerSmaI(co) cleaved with SmaI using In-Fusion HD cloning kit to produce pwAup-DN H+L+J(co).

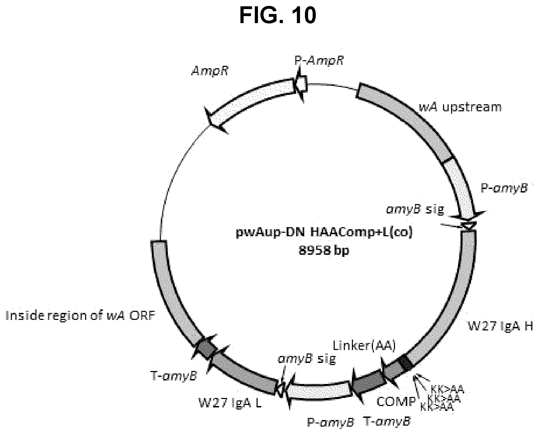

For transformation to obtain pentamer IgA expressing DNA, a donor DNA plasmid pwAup-DN HAAComp+L(co) (Fig. 10) was produced as follows.

The signal sequence of AmyB and the coding regions of the H chain and C-terminal linker of W27 IgA were amplified. A DNA fragment containing a part of a linker sequence, a COMP domain, an amyB terminator, an amyB promoter, a signal sequence of AmyB, and a coding region of an L chain of W27 IgA was amplified. At the time of amplification of these DNA fragments, the lysine residue originally contained in the linker connecting the H chain and the COMP domain was substituted with alanine. These two DNA fragments were connected by plasmid pwAupADN cleaved with SmaI using In-Fusion HD cloning kit to produce pwAup-DN HAAComp+L(co).

### 4. Obtaining of IgA-Producing Strain by Transformation of A. oryzae

For transformation to obtain a dimeric IgA-producing strain, a donor DNA plasmid pwAup-DN H+L+J(co) (Fig. 1) and a genome editing plasmid pRGE-gwAup (Fig. 2) were used. For transformation to obtain a pentamer IgA-producing strain, a donor DNA plasmid pwAup-DN HAAComp+L(co) (Fig. 10) and a genome editing plasmid pRGE-gwAup (Fig. 2) were used. As a host for transformation, the 10 protease genes disrupted strains NSlD1-ΔP10 [Yoon et al. Appl Microbiol Biotechnol. 89:747-759, 2011] was used. Transformation of A. oryzae was performed according to the method described in the previous report [Maruyama et al., Methods Mol Biol 765:447-456, 2011].

### 5. Production of W27 IgA and Ammonium Sulfate Precipitation

To produce an IgA antibody, 5 x DPY medium (10% Dextrin, 5% Hipolypeptone, 2.5% Yeast extract, 0.5% K₂HPO₄, 0.05% MgSO₄·7H₂O, pH 8.0) or DPY medium (2% Dextrin, 1% Hipolypeptone, 0.5% Yeast extract, 0.5% K₂HPO₄, 0.05% MgSO₄·7H₂O, pH 8.0) was used. Conidia of an IgA-producing strain were inoculated into a liquid medium so as to be 1 × 10⁷ per 100 mL, and shake-cultured at 150 rpm and 30°C for 8 days. The culture supernatant was collected by filtration with Miracloth (Merck Millipore). The amount of IgA produced in the culture solution of the transformant was analyzed by ELISA assay or Western analysis.

### 6. Concentration of Produced IgA by Ammonium Sulfate Precipitation

A concentration of the produced IgA by ammonium sulfate precipitation was performed as follows. The culture supernatant obtained by filtration using Miracloth (Merck Millipore) was centrifuged at 10,000 × g and 4°C for 10 minutes in the case of culture with 5 × DPY medium, or at 3,500 rpm (2,150 × g) and 4°C for 10 minutes in the case of using DPY medium, and the supernatant was collected. The pH was adjusted by adding 1/10 volume of 10 × PBS buffer, and further diluted 5 times with PBS buffer in culture with 5 × DPY medium. 390 g of (NH₄)₂SO₄ (FUJIFILM Wako Chemicals) was added per 1 L at 60% saturation and the mixture was incubated overnight at 4°C with gentle stirring. Thereafter, the mixture was centrifuged at 10,000 × g and 4°C for 20 minutes to obtain a precipitate, a small amount of a 60% saturated (NH₄)₂SO₄ solution was added, and the mixture was washed by centrifugation at 10,000 × g and 4°C for 10 minutes. The precipitate was dissolved in PBS buffer and dialyzed against PBS buffer using a 14,000 MWCO dialysis membrane (Wako Pure Chemical Industries, Ltd.) for 24 hours. The PBS buffer was exchanged twice on the way. After dialysis, the mixture was centrifuged at 15,000 rpm (20,620 × g) and 4°C for 10 minutes in culture with 5 × DPY medium, or at 3,500 rpm (2,150 × g) and 4°C for 10 minutes in the case of using DPY medium, and the supernatant was sequentially filtered through filters of 3.0 µm, 0.45 µm, and 0.2 µm.

### 7. Western Analysis of W27 IgA

Western analysis of samples containing the produced IgA was performed using the following antibodies.

For detection of the H chain, an anti-H chain antibody Goat Anti-Mouse IgA-UNLB (Cat. No. 1040-01, SouthernBiotech), a secondary antibody Rabbit anti-goat IgG(H+L)/(H&L) antibody, and an HRP conjugate (Cat. No. SA00001-4, Proteintech Group, Inc.) were used.

For detection of the L chain, an anti-L chain antibody Goat Anti-Mouse Kappa-UNLB (Cat. No. 1050-01, SouthernBiotech), a secondary antibody Rabbit anti-goat IgG(H+L)/(H&L) antibody, and an HRP conjugate (Cat. No. SA00001-4, Proteintech Group, Inc.) were used.

For detection of the J chain, an anti-J chain antibody Ig J chain (M-159) (Cat. No. sc-66929, Santa Curz Biotechnology) and a secondary antibody Anti-rabbit IgG, HRP-linked antibody (Cat. No. 7074, Cell Signaling Technology) were used.

### 8. Quantification of Monoclonal IgA Antibody and Non-Reduced SDS-PAGE

Quantification of the purified antibody was performed by an ELISA method. Details of the antibody quantification method are as follows. First, Anti-goat-mouse IgA (Southern Biotech: 1040-01) was diluted with 0.05 M Na₂CO₃ to 2 µg/ml, added to each well of C96 MaxiSorp Nunc-Immuno Plate (Thermo Fisher) (hereinafter, referred to as ELISA plate) by 50 µl/well, and immobilized overnight at 4°C. The plate was washed 3 times with PBS using Vaccu-Pette/96 multi-well pipetter (Sigma-Aldrich) (hereinafter, plate washer), then PBS (PBS-BSA) containing 1% bovine serum albumin (BSA) (Wako) was added so as to be 150 µl/well, and the plate was allowed to stand at 4°C overnight to perform blocking. A series of antibody fluids was prepared in a 96-well plate. A dilution series of the antibody fluids was prepared using PBS-BSA at equal magnification (x1, x1/100 and x1/1,000 of the purified antibodies are defined as x1), 3-fold (x1/3), 10-fold (x1/10), 30-fold (x1/30), 100-fold (x1/100), 300-fold (x1/300), 1,000-fold (x1/1,000), and 3,000-fold (x1/3,000). After PBS-BSA was completely removed from the plate, the serial dilution was added so as to be 50 µl/well, and the plate was allowed to stand at room temperature for 1 hour for reaction.

Next, the plate was washed 3 times with a plate washer with PBS (PBS-T) containing 0.05%Tween 20 (Chem Cruz), and then, a secondary antibody Alkaline Phosphatase (ALP)-conjugated anti-goat mouse IgA (Southern Biotech: 1040-04) was diluted with PBS-BSA to a final concentration of 0.5 µg/ml, added at 50 µl/well, and allowed to stand at room temperature for 1 hour for reaction. The plate was washed 3 times with a plate washer using PBS-T, and then a substrate solution prepared by adding 10 µl of 1 M MgCl₂ and 1 tablet of Substrate for Alkaline Phosphatase (Sigma-Aldrich) to 5 ml of a carbonate buffer (6.5 mM Na₂CO₃, 18.5 mM NaHCO₃) was added at 50 µl/well. After the mixture was left to stand while being shielded from light at room temperature, it was visually confirmed that the color was sufficiently developed, and absorbance at an optical density (O.D.) of 405 nm was measured using TriStar² LB942 (BERTHOLD TECHNOLOGIES).

Apart from the above, whether a dimeric antibody was produced or not was confirmed by non-reduced SDS-PAGE. For sample preparation, 3 × Native buffer (0.2 M Tris-HCl pH 6.8, 30% Glycerol, 0.015% Bromophenol blue) was added to the antibody (5 µg). Using 6% SDS-PAGE gel using WIDE RANGE Gel Preparation Buffer (4×) for PAGE (Nacalai Tesque), electrophoresis was performed, and then, Coomassie brilliant blue (CBB) staining was performed. In the reduced SDS-PAGE, a 2-ME-added buffer was added to a sample, the sample was denatured at 95°C for 10 minutes, the sample was electrophoresed using 6% SDS-PAGE gel as in the case of non-reduction, and then, Coomassie brilliant blue (CBB) staining was performed.

### 9. Analysis of Binding Force of Purified IgA Antibody to E. coli

In order to compare the binding force of the purified IgA antibody to E. coli, ELISA to E. coli was performed. E. coli (DH5α or BW38092 and a variant thereof, all of which are K12 strains) was subjected to aerobic culture (LB medium) at 37°C overnight and collected by centrifugation. After washing the bacterial cell body with PBS, bacteria were suspended in a 0.05 M Na₂CO₃ buffer and coated onto an ELISA plate (4°C overnight). As in the purified IgA antibody quantitative ELISA, after blocking with PBS containing 1% BSA, each antibody dilution series in a 96-well plate was prepared and added, and reacted at room temperature for about 1 hour. Thereafter, the plate was washed with 0.05% Tween 20-added PBS, and a secondary antibody Alkaline Phosphatase (ALP)-conjugated anti-goat mouse IgA (Southern Biotech: 1040-04) was diluted with PBS-BSA to a final concentration of 0.5 µg/ml, added at 50 µl/well, and allowed to stand at room temperature for 1 hour for reaction. After washing the plate three times with a plate washer with PBS-T, a color developing reaction was performed in the same manner as described above using Alkali Phosphatase tablet (Sigma). For sufficient reaction, the ELISA plate after color development was reacted at 4 degrees overnight, and OD₄₀₅ nm was measured using TriStar² LB942 (BERTHOLD TECHNOLOGIES).

### 10. E. coli and C. difficile Growth Inhibitory Test (Anaerobic)

E. coli was anaerobically cultured in LB medium (37°C overnight). All subsequent manipulations were performed in an anaerobic chamber. The culture solution was centrifuged, and bacteria were collected and then washed with LB medium. After the bacterial solution was diluted 10 times with a culture solution, SYTO24 (Invitrogen) and Counting beads contained in Cell Viability Kit (Becton Dickinson) were added, and the number of SYTO24 positive viable bacteria was calculated by flow cytometry (measurement was under aerobic conditions) from comparison with the Counting beads. The bacterial solution was diluted with LB medium to 300 cells/5 µl. 5 µl of the bacterial diluted solution was added to an Eppendorf tube, 25 µl of anaerobized PBS or anaerobized purified antibody was further added, 30 µl of LB medium was further added, and static culture was performed anaerobically at 37°C (final concentration of antibody was 0.42 mg/ml). Thereafter, the bacterial solution was diluted for 20 hours and seeded on an LB plate, and anaerobically cultured at 37°C overnight. On the next day, the number of colonies was measured to determine the number of viable bacteria.

In the same manner as in the test for E. coli, 10,000 cells of C. difficile were anaerobically cultured together with a multimeric W27 IgA antibody (417 mg/ml) obtained from a dimeric IgA-expression-modified koji strain (KojiHLJ#1) at 37°C for 20 hours, and then seeded on a medium plate (LB plate) for E. coli, and the number of colonies was measured.

### Example 1: Production of Dimeric IgA-Producing Strain

In A. oryzae, it was attempted to produce a W27 IgA antibody into a dimer. A dimer of IgA is composed of an H chain, an L chain, and a J chain. For the genes encoding each, codon modification was performed based on the codon use frequency of tef1, which is one of the genes having the highest expression level in A. oryzae. On the other hand, a sequence encoding a signal sequence of α-amylase AmyB, which is secreted in the largest amount in A. oryzae, was ligated and designed to be translocated to the endoplasmic reticulum and secreted after translation. As the promoter and the terminator, those of the amyB gene were used (Fig. 1).

For gene introduction, a genetic modification technology by the genome editing technology CRISPR/Cas9 system in A. oryzae [Katayama et al., Appl. Environ. Microbiol., 85:e01896-18, 2019] was used. As the IgA antibody, based on the W27 antibody described in WO 2014/142084 A and JP 2021-110421 A information, RS_H000_L001, which is a CHO cell producing recombinant IgA of the W27 antibody, in which a mutation for protein L binding was introduced into the light chain, was used. The amino acid sequences of the heavy chain variable region and the light chain variable region of RS_H000_L001 are shown below. Hereinafter, in Examples 1 to 4, RS_H000_L001 is referred to as "'W27 IgA".

| | |
|---|---|
| Heavy chain variable region SEQ ID NO: 1 (W27G2_HV) | MKCSWIIFFLMAVVTGVNSEVQLQQSGSELVKSGASVKLSCTVS GFNFTDYYIHWVRQRTEQGLEWIGRIDPENDETTYAPKFQGKAT MTADTSSNTAYLQLTSLTSEDTAVYYCARSTVLDYWGHGTTLTV SS |
| Light chain variable region SEQ ID NO: 2 (RS_LV001) | MKLPVRLLVLMFWIPGFSSDVLMTQSPASLSVSLGDRATISCRA SQSIVHTNGNTYLEWYLQKPGQSPKLLIYKVSNRFSGVPDRFSG SGSGTDFILKISRVEAEDLGVYYCFQGSHVPPTFGGGTKLEVK |

Note that, although the above sequence includes a signal sequence derived from a mouse, W27 IgA used for genome modification of a filamentous fungus in the present example contains a signal sequence of α-amylase AmyB as described below.

Cassettes expressing the H chain, L chain, and J chain of W27 IgA antibody were connected in tandem. Such an expression cassette was designed to be inserted into the wA locus by homologous recombination by being positioned inside the upstream region and the ORF internal region of the dye synthesis gene wA, thereby producing a donor DNA plasmid pwAup-DN H+L+J(co) (Fig. 1).

The genome editing plasmid pRGE-gwAup [Katayama et al., Appl. Environ. Microbiol., 85:e01896-18, 2019] was used to express sgRNA performing double-stranded DNA cleavage targeting the upstream of the wA gene (Fig. 2).

As a host for transformation of A. oryzae, the 10 protease genes disrupted strains NSlD1-ΔP10 [Yoon et al., Appl. Microbiol. Biotechnol., 89:747-759, 2011] was used. It has been shown that the use of the strain increases the production amount of human-derived lysozymes, bovine-derived chymosins [Yoon et al., Appl. Microbiol. Biotechnol., 89:747-759, 2011], and human-derived IgG antibodies [Huynh et al., Fungal Biol. Biotechnol., 7:7, 2020].

The NSlD1-ΔP10 strain was transformed using the donor DNA plasmid pwAup-DN H+L+J(co) for dimeric IgA expression and the genome editing plasmid pRGE-gwAup. At this time, based on the previous report [Katayama et al., Appl. Environ. Microbiol., 85:e01896-18, 2019], the amount of the donor DNA plasmid used for the genome editing plasmid was increased, such that a transformant into which multiple copies of the expression cassette were introduced was easily obtained. For the resulting transformed strain, the copy number and the production amount of the expression cassette of dimeric IgA were analyzed, and a strain having a high copy number and a high production amount was named as "H+L+J(co) #1" and used for the subsequent production experiment.

The dimeric IgA-producing strain "H+L+J(co) #1" was inoculated into a liquid medium and cultured for 8 days, and then, the culture supernatant was collected and concentrated to about 10 times by ammonium sulfate precipitation. As a result of Western analysis, the H chain, the L chain, and the J chain were detected in the culture supernatant and the concentrated sample, and production to the culture solution was confirmed (Fig. 3).

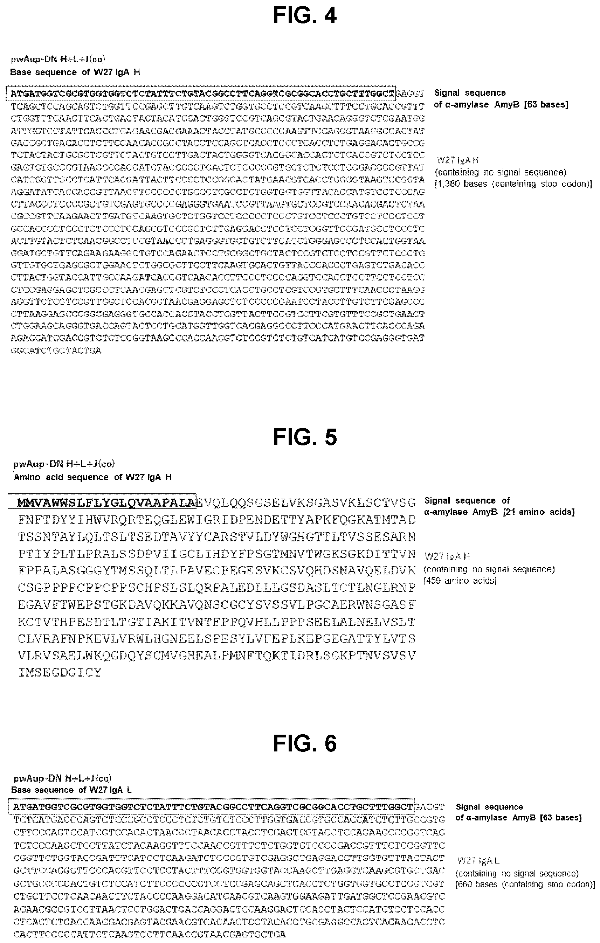

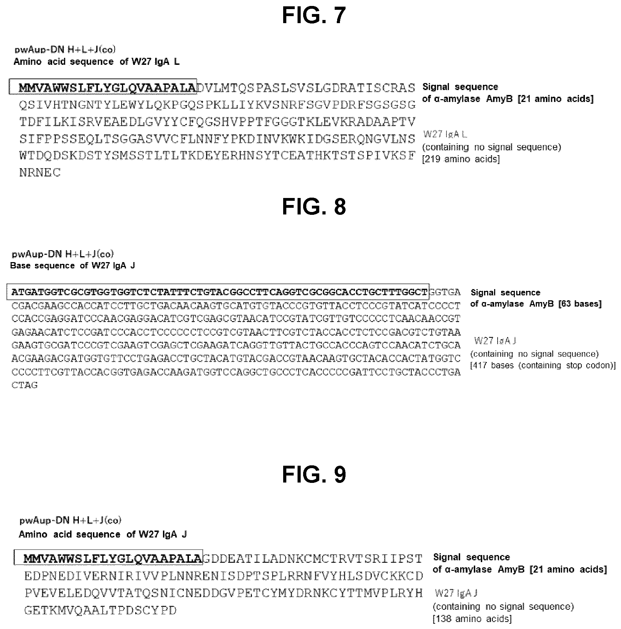

The nucleic acid sequences and amino acid sequences of W27 IgA H (heavy chain), W27 IgA L (light chain), and W27 IgA J (J chain) contained in the plasmid pwAup-DN H+L+J(co) are illustrated in Figs. 4 to 9.

> SEQ ID NO: 3: Base sequence of W27 IgA H contained in pwAup-DN H+L+J(co) (containing signal sequence of α-amylase AmyB) 
ATGATGGTCGCGTGGTGGTCTCTATTTCTGTACGGCCTTCAGGTCGCGGCACCTGCTTT
GGCTGAGGTTCAGCTCCAGCAGTCTGGTTCCGAGCTTGTCAAGTCTGGTGCCTCCGTCA
AGCTTTCCTGCACCGTTTCTGGTTTCAACTTCACTGACTACTACATCCACTGGGTCCGT
CAGCGTACTGAACAGGGTCTCGAATGGATTGGTCGTATTGACCCTGAGAACGACGAAAC
TACCTATGCCCCCAAGTTCCAGGGTAAGGCCACTATGACCGCTGACACCTCTTCCAACA
CCGCCTACCTCCAGCTCACCTCCCTCACCTCTGAGGACACTGCCGTCTACTACTGCGCT
CGTTCTACTGTCCTTGACTACTGGGGTCACGGCACCACTCTCACCGTCTCCTCCGAGTC
TGCCCGTAACCCCACCATCTACCCCCTCACTCTCCCCCGTGCTCTCTCCTCCGACCCCG
TTATCATCGGTTGCCTCATTCACGATTACTTCCCCTCCGGCACTATGAACGTCACCTGG
GGTAAGTCCGGTAAGGATATCACCACCGTTAACTTCCCCCCTGCCCTCGCCTCTGGTGG
TGGTTACACCATGTCCTCCCAGCTTACCCTCCCCGCTGTCGAGTGCCCCGAGGGTGAAT
CCGTTAAGTGCTCCGTCCAACACGACTCTAACGCCGTTCAAGAACTTGATGTCAAGTGC
TCTGGTCCTCCCCCTCCCTGTCCTCCCTGTCCTCCCTCCTGCCACCCCTCCCTCTCCCT
CCAGCGTCCCGCTCTTGAGGACCTCCTCCTCGGTTCCGATGCCTCCCTCACTTGTACTC
TCAACGGCCTCCGTAACCCTGAGGGTGCTGTCTTCACCTGGGAGCCCTCCACTGGTAAG
GATGCTGTTCAGAAGAAGGCTGTCCAGAACTCCTGCGGCTGCTACTCCGTCTCCTCCGT
TCTCCCTGGTTGTGCTGAGCGCTGGAACTCTGGCGCTTCCTTCAAGTGCACTGTTACCC
ACCCTGAGTCTGACACCCTTACTGGTACCATTGCCAAGATCACCGTCAACACCTTCCCT
CCCCAGGTCCACCTCCTTCCTCCTCCCTCCGAGGAGCTCGCCCTCAACGAGCTCGTCTC
CCTCACCTGCCTCGTCCGTGCTTTCAACCCTAAGGAGGTTCTCGTCCGTTGGCTCCACG
GTAACGAGGAGCTCTCCCCCGAATCCTACCTTGTCTTCGAGCCCCTTAAGGAGCCCGGC
GAGGGTGCCACCACCTACCTCGTTACTTCCGTCCTTCGTGTTTCCGCTGAACTCTGGAA
GCAGGGTGACCAGTACTCCTGCATGGTTGGTCACGAGGCCCTTCCCATGAACTTCACCC
AGAAGACCATCGACCGTCTCTCCGGTAAGCCCACCAACGTCTCCGTCTCTGTCATCATG
TCCGAGGGTGATGGCATCTGCTACTGA
> SEQ ID NO: 4: Amino acid sequence of W27 IgA H contained in pwAup-DN H+L+J (co) (containing signal sequence of α-amylase AmyB) MMVAWWSLFLYGLQVAAPALAEVQLQQSGSELVKSGASVKLSCTVSGFNFTDYYIHW 
VRQRTEQGLEWIGRIDPENDETTYAPKFQGKATMTADTSSNTAYLQLTSLTSEDTAV
YYCARSTVLDYWGHGTTLTVSSESARNPTIYPLTLPRALSSDPVIIGCLIHDYFPSG
TMNVTWGKSGKDITTVNFPPALASGGGYTMSSQLTLPAVECPEGESVKCSVQHDSNA
VQELDVKCSGPPPPCPPCPPSCHPSLSLQRPALEDLLLGSDASLTCTLNGLRNPEGA
VFTWEPSTGKDAVQKKAVQNSCGCYSVSSVLPGCAERWNSGASFKCTVTHPESDTLT
GTIAKITVNTFPPQVHLLPPPSEELALNELVSLTCLVRAFNPKEVLVRWLHGNEELS
PESYLVFEPLKEPGEGATTYLVTSVLRVSAELWKQGDQYSCMVGHEALPMNFTQKTI
DRLSGKPTNVSVSVIMSEGDGICY*
> SEQ ID NO: 5: Base sequence of W27 IgA L contained in pwAup-DN H+L+J(co) (containing signal sequence of α-amylase AmyB), 
ATGATGGTCGCGTGGTGGTCTCTATTTCTGTACGGCCTTCAGGTCGCGGCACCTGCT
TTGGCTGACGTTCTCATGACCCAGTCTCCCGCCTCCCTCTCTGTCTCCCTTGGTGAC
CGTGCCACCATCTCTTGCCGTGCTTCCCAGTCCATCGTCCACACTAACGGTAACACC
TACCTCGAGTGGTACCTCCAGAAGCCCGGTCAGTCTCCCAAGCTCCTTATCTACAAG
GTTTCCAACCGTTTCTCTGGTGTCCCCGACCGTTTCTCCGGTTCCGGTTCTGGTACC
GATTTCATCCTCAAGATCTCCCGTGTCGAGGCTGAGGACCTTGGTGTTTACTACTGC
TTCCAGGGTTCCCACGTTCCTCCTACTTTCGGTGGTGGTACCAAGCTTGAGGTCAAG
CGTGCTGACGCTGCCCCCACTGTCTCCATCTTCCCCCCCTCCTCCGAGCAGCTCACC
TCTGGTGGTGCCTCCGTCGTCTGCTTCCTCAACAACTTCTACCCCAAGGACATCAAC
GTCAAGTGGAAGATTGATGGCTCCGAACGTCAGAACGGCGTCCTTAACTCCTGGACT
GACCAGGACTCCAAGGACTCCACCTACTCCATGTCCTCCACCCTCACTCTCACCAAG
GACGAGTACGAACGTCACAACTCCTACACCTGCGAGGCCACTCACAAGACCTCCACT
TCCCCCATTGTCAAGTCCTTCAACCGTAACGAGTGCTGA
> SEQ ID NO: 6: Amino acid sequence of W27 IgA L contained in pwAup-DN H+L+J(co), (containing signal sequence of α-amylase AmyB) 
MMVAWWSLFLYGLQVAAPALADVLMTQSPASLSVSLGDRATISCRASQSIVHTNGNT
YLEWYLQKPGQSPKLLIYKVSNRFSGVPDRFSGSGSGTDFILKISRVEAEDLGVYYC
FQGSHVPPTFGGGTKLEVKRADAAPTVSIFPPSSEQLTSGGASVVCFLNNFYPKDIN
VKWKIDGSERQNGVLNSWTDQDSKDSTYSMSSTLTLTKDEYERHNSYTCEATHKTST
SPIVKSFNRNEC*
> SEQ ID NO: 7: Base sequence of W27 IgA J contained in pwAup-DN H+L+J(co) (containing signal sequence of α-amylase AmyB), 
ATGATGGTCGCGTGGTGGTCTCTATTTCTGTACGGCCTTCAGGTCGCGGCACCTGCT
TTGGCTGGTGACGACGAAGCCACCATCCTTGCTGACAACAAGTGCATGTGTACCCGT
GTTACCTCCCGTATCATCCCCTCCACCGAGGATCCCAACGAGGACATCGTCGAGCGT
AACATCCGTATCGTTGTCCCCCTCAACAACCGTGAGAACATCTCCGATCCCACCTCC
CCCCTCCGTCGTAACTTCGTCTACCACCTCTCCGACGTCTGTAAGAAGTGCGATCCC
GTCGAAGTCGAGCTCGAAGATCAGGTTGTTACTGCCACCCAGTCCAACATCTGCAAC
GAAGACGATGGTGTTCCTGAGACCTGCTACATGTACGACCGTAACAAGTGCTACACC
ACTATGGTCCCCCTTCGTTACCACGGTGAGACCAAGATGGTCCAGGCTGCCCTCACC
CCCGATTCCTGCTACCCTGACTAG
> SEQ ID NO: 8: Amino acid sequence of W27 IgA J contained in pwAup-DN H+L+J (co) (containing signal sequence of α-amylase AmyB)

MMVAWWSLFLYGLQVAAPALAGDDEATILADNKCMCTRVTSRIIPSTEDPNEDIVER
NIRIVVPLNNRENISDPTSPLRRNFVYHLSDVCKKCDPVEVELEDQVVTATQSNICN
EDDGVPETCYMYDRNKCYTTMVPLRYHGETKMVQAALTPDSCYPD*

### Example 2: Production of Pentamer IgA-Producing Strain

In order to produce a W27 IgA antibody into a pentamer, a COMP domain was used. The cartilage oligomeric matrix protein (COMP) domain is a coiled-coil domain derived from a cartilage oligomeric matrix protein, and has been used for inducing pentamer formation.

For gene introduction, a genetic modification technology by the genome editing technology CRISPR/Cas9 system in A. oryzae [Katayama et al., Appl. Environ. Microbiol., 85:e01896-18, 2019] was used. The COMP domain was fused to the C-terminal side of the H chain of the W27 IgA antibody, and a cassette for expression together with the L chain was connected in tandem. At this time, the lysine residue originally contained in the linker connecting the H chain and the COMP domain was substituted with alanine. Such an expression cassette, using the promoter and terminator of the amyB gene, was designed to be inserted into the wA locus by homologous recombination by being positioned inside the upstream region and the ORF internal region of the dye synthesis gene wA, thereby producing a donor DNA plasmid pwAup-DN HAAComp+L(co) (Fig. 10). The genome editing plasmid pRGE-gwAup [Katayama et al., Appl. Environ. Microbiol., 85:e01896-18, 2019] was used to express sgRNA performing double-stranded DNA cleavage targeting the upstream of the wA gene.

The NSlD1-ΔP10 strain was transformed using the produced donor DNA plasmid pwAup-DN HAAComp+L(co) and the genome editing plasmid pRGE-gwAup. At this time, based on the previous report [Katayama et al., Appl. Environ. Microbiol., 85:e01896-18, 2019], the amount of the donor DNA plasmid used for the genome editing plasmid was increased, such that a transformant into which multiple copies of the expression cassette were introduced was easily obtained. For the resulting transformed strain, the copy number and the production amount of the expression cassette of pentamer IgA were analyzed, and a strain having a high copy number and a high production amount was named as "HAAComp+L(co) #1" and used for the subsequent production experiment.

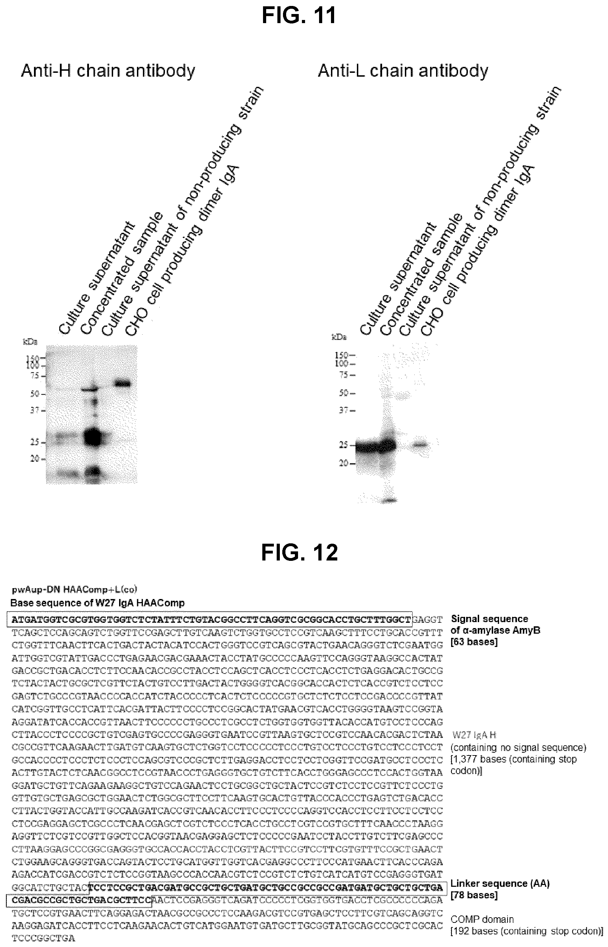

The pentamer IgA-producing strain "HAAComp+L(co) #1" was inoculated into a liquid medium and cultured for 8 days, and then, the culture supernatant was collected and concentrated to about 10 times by ammonium sulfate precipitation. As a result of Western analysis, the H chain and the L chain were detected in the culture supernatant and the concentrated sample, and production to the culture solution was confirmed (Fig. 11).

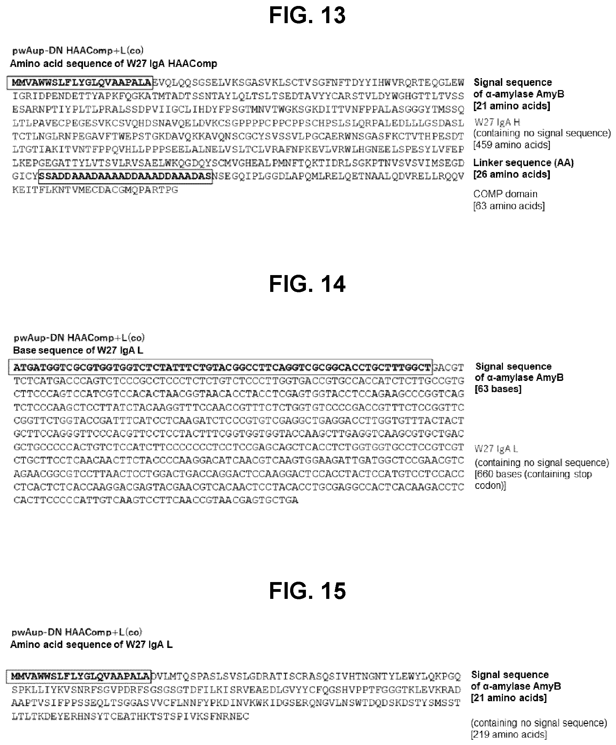

The nucleic acid sequences and amino acid sequences of W27 IgA H (heavy chain) and W27 IgA L (light chain) contained in the plasmid pwAup-DN HAAComp+L(co) are illustrated in Figs. 12 to 15.

> SEQ ID NO: 9: Base sequence of W27 IgA H contained in pwAup-DN HAAComp+L (co) (containing signal sequence of α-amylase AmyB, linker sequence, and COMP sequence) 
ATGATGGTCGCGTGGTGGTCTCTATTTCTGTACGGCCTTCAGGTCGCGGCACCTGCT
TTGGCTGAGGTTCAGCTCCAGCAGTCTGGTTCCGAGCTTGTCAAGTCTGGTGCCTCC
GTCAAGCTTTCCTGCACCGTTTCTGGTTTCAACTTCACTGACTACTACATCCACTGG
GTCCGTCAGCGTACTGAACAGGGTCTCGAATGGATTGGTCGTATTGACCCTGAGAAC
GACGAAACTACCTATGCCCCCAAGTTCCAGGGTAAGGCCACTATGACCGCTGACACC
TCTTCCAACACCGCCTACCTCCAGCTCACCTCCCTCACCTCTGAGGACACTGCCGTC
TACTACTGCGCTCGTTCTACTGTCCTTGACTACTGGGGTCACGGCACCACTCTCACC
GTCTCCTCCGAGTCTGCCCGTAACCCCACCATCTACCCCCTCACTCTCCCCCGTGCT
CTCTCCTCCGACCCCGTTATCATCGGTTGCCTCATTCACGATTACTTCCCCTCCGGC
ACTATGAACGTCACCTGGGGTAAGTCCGGTAAGGATATCACCACCGTTAACTTCCCC
CCTGCCCTCGCCTCTGGTGGTGGTTACACCATGTCCTCCCAGCTTACCCTCCCCGCT
GTCGAGTGCCCCGAGGGTGAATCCGTTAAGTGCTCCGTCCAACACGACTCTAACGCC
GTTCAAGAACTTGATGTCAAGTGCTCTGGTCCTCCCCCTCCCTGTCCTCCCTGTCCT
CCCTCCTGCCACCCCTCCCTCTCCCTCCAGCGTCCCGCTCTTGAGGACCTCCTCCTC
GGTTCCGATGCCTCCCTCACTTGTACTCTCAACGGCCTCCGTAACCCTGAGGGTGCT
GTCTTCACCTGGGAGCCCTCCACTGGTAAGGATGCTGTTCAGAAGAAGGCTGTCCAG
AACTCCTGCGGCTGCTACTCCGTCTCCTCCGTTCTCCCTGGTTGTGCTGAGCGCTGG
AACTCTGGCGCTTCCTTCAAGTGCACTGTTACCCACCCTGAGTCTGACACCCTTACT
GGTACCATTGCCAAGATCACCGTCAACACCTTCCCTCCCCAGGTCCACCTCCTTCCT
CCTCCCTCCGAGGAGCTCGCCCTCAACGAGCTCGTCTCCCTCACCTGCCTCGTCCGT
GCTTTCAACCCTAAGGAGGTTCTCGTCCGTTGGCTCCACGGTAACGAGGAGCTCTCC
CCCGAATCCTACCTTGTCTTCGAGCCCCTTAAGGAGCCCGGCGAGGGTGCCACCACC
TACCTCGTTACTTCCGTCCTTCGTGTTTCCGCTGAACTCTGGAAGCAGGGTGACCAG
 
TACTCCTGCATGGTTGGTCACGAGGCCCTTCCCATGAACTTCACCCAGAAGACCATC
GACCGTCTCTCCGGTAAGCCCACCAACGTCTCCGTCTCTGTCATCATGTCCGAGGGT
GATGGCATCTGCTACTCCTCCGCTGACGATGCCGCTGCTGATGCTGCCGCCGCCGAT
GATGCTGCTGCTGACGACGCCGCTGCTGACGCTTCCAACTCCGAGGGTCAGATCCCC
CTCGGTGGTGACCTCGCCCCCCAGATGCTCCGTGAACTTCAGGAGACTAACGCCGCC
CTCCAAGACGTCCGTGAGCTCCTTCGTCAGCAGGTCAAGGAGATCACCTTCCTCAAG
AACACTGTCATGGAATGTGATGCTTGCGGTATGCAGCCCGCTCGCACTCCCGGCTGA
> SEQ ID NO: 10: Amino acid sequence of W27 IgA H contained in pwAup-DN HAAComp+L(co) (containing signal sequence of α-amylase AmyB, linker sequence, and COMP sequence) 
MMVAWWSLFLYGLQVAAPALAEVQLQQSGSELVKSGASVKLSCTVSGFNFTDYYIHW
VRQRTEQGLEWIGRIDPENDETTYAPKFQGKATMTADTSSNTAYLQLTSLTSEDTAV
YYCARSTVLDYWGHGTTLTVSSESARNPTIYPLTLPRALSSDPVIIGCLIHDYFPSG
TMNVTWGKSGKDITTVNFPPALASGGGYTMSSQLTLPAVECPEGESVKCSVQHDSNA
VQELDVKCSGPPPPCPPCPPSCHPSLSLQRPALEDLLLGSDASLTCTLNGLRNPEGA
VFTWEPSTGKDAVQKKAVQNSCGCYSVSSVLPGCAERWNSGASFKCTVTHPESDTLT
GTIAKITVNTFPPQVHLLPPPSEELALNELVSLTCLVRAFNPKEVLVRWLHGNEELS
PESYLVFEPLKEPGEGATTYLVTSVLRVSAELWKQGDQYSCMVGHEALPMNFTQKTI
DRLSGKPTNVSVSVIMSEGDGICYSSADDAAADAAADDAAADDAAADASNSEGQIP
LGGDLAPQMLRELQETNAALQDVRELLRQQVKEITFLKNTVMECDACGMQPARTPG*
> SEQ ID NO: 11: Base sequence of W27 IgA L contained in pwAup-DN HAAComp+L (co) (containing signal sequence of α-amylase AmyB) 
ATGATGGTCGCGTGGTGGTCTCTATTTCTGTACGGCCTTCAGGTCGCGGCACCTGCT
TTGGCTGACGTTCTCATGACCCAGTCTCCCGCCTCCCTCTCTGTCTCCCTTGGTGAC
CGTGCCACCATCTCTTGCCGTGCTTCCCAGTCCATCGTCCACACTAACGGTAACACC
TACCTCGAGTGGTACCTCCAGAAGCCCGGTCAGTCTCCCAAGCTCCTTATCTACAAG
GTTTCCAACCGTTTCTCTGGTGTCCCCGACCGTTTCTCCGGTTCCGGTTCTGGTACC
GATTTCATCCTCAAGATCTCCCGTGTCGAGGCTGAGGACCTTGGTGTTTACTACTGC
TTCCAGGGTTCCCACGTTCCTCCTACTTTCGGTGGTGGTACCAAGCTTGAGGTCAAG
CGTGCTGACGCTGCCCCCACTGTCTCCATCTTCCCCCCCTCCTCCGAGCAGCTCACC
TCTGGTGGTGCCTCCGTCGTCTGCTTCCTCAACAACTTCTACCCCAAGGACATCAAC
GTCAAGTGGAAGATTGATGGCTCCGAACGTCAGAACGGCGTCCTTAACTCCTGGACT
GACCAGGACTCCAAGGACTCCACCTACTCCATGTCCTCCACCCTCACTCTCACCAAG
GACGAGTACGAACGTCACAACTCCTACACCTGCGAGGCCACTCACAAGACCTCCACT
TCCCCCATTGTCAAGTCCTTCAACCGTAACGAGTGCTGA
> SEQ ID NO: 12: Amino acid sequence of W27 IgA L contained in pwAup-DN HAAComp+L(co) (containing signal sequence of α-amylase AmyB)

MMVAWWSLFLYGLQVAAPALADVLMTQSPASLSVSLGDRATISCRASQSIVHTNGNT
YLEWYLQKPGQSPKLLIYKVSNRFSGVPDRFSGSGSGTDFILKISRVEAEDLGVYYC
FQGSHVPPTFGGGTKLEVKRADAAPTVSIFPPSSEQLTSGGASVVCFLNNFYPKDIN

VKWKIDGSERQNGVLNSWTDQDSKDSTYSMSSTLTLTKDEYERHNSYTCEATHKTST
SPIVKSFNRNEC*

### Example 3: Influence of Configuration of IgA Fusion Protein

For a fusion protein obtained by adding a COMP sequence to the C-terminus of the H chain of IgA, the influence of the configuration of the H chain C-terminal region of IgA and the configuration of the linker on multimer formation was examined.

Genome-modified CHO cells were produced using a nucleic acid construct encoding IgA containing a natural L chain and a fusion protein having the following configuration as an H chain, and the multimer formation state of secreted IgA was examined.

| Configuration name | Configuration of H chain | Amino acid sequence name of H chain/SEQ ID NO: |
|---|---|---|
| AAcomp | COMP sequence is fused to C-terminus of full-length H chain via linker consisting of amino acid sequence SSADDAAADAAAADDAAADDAAADAS | SEQ ID NO: 13 |
| KKcomp | COMP sequence is fused to C-terminus of full-length H chain via linker consisting of amino acid sequence SSADDAKKDAAKKDDAKKDDAKKDAS | SEQ ID NO: 14 |
| direct-comp | COMP sequence is fused to C-terminus of full-length H chain without linker | SEQ ID NO: 15 |
| notail-comp | COMP sequence is fused to C-terminus of H chain lacking tail piece without linker | SEQ ID NO: 16 |

> SEQ ID NO: 13: AAcomp W27 IgA H amino acid sequence (without signal sequence) 
EVQLQQSGSELVKSGASVKLSCTVSGFNFTDYYIHWVRQRTEQGLEWIGRIDPENDETT
YAPKFQGKATMTADTSSNTAYLQLTSLTSEDTAVYYCARSTVLDYWGHGTTLTVSSESA
RNPTIYPLTLPRALSSDPVIIGCLIHDYFPSGTMNVTWGKSGKDITTVNFPPALASGGG
YTMSSQLTLPAVECPEGESVKCSVQHDSNAVQELDVKCSGPPPPCPPCPPSCHPSLSLQ
RPALEDLLLGSDASLTCTLNGLRNPEGAVFTWEPSTGKDAVQKKAVQNSCGCYSVSSVL
PGCAERWNSGASFKCTVTHPESDTLTGTIAKITVNTFPPQVHLLPPPSEELALNELVSL
TCLVRAFNPKEVLVRWLHGNEELSPESYLVFEPLKEPGEGATTYLVTSVLRVSAELWKQ
GDQYSCMVGHEALPMNFTQKTIDRLSGKPTNVSVSVIMSEGDGICYSSADDAAADAAA
 
DDAAADDAAADASNSEGQIPLGGDLAPQMLRELQETNAALQDVRELLRQQVKEITFLKN
TVMECDACGMQPARTPG
> SEQ ID NO: 14: KKcomp W27 IgA H amino acid sequence (without signal sequence) 
EVQLQQSGSELVKSGASVKLSCTVSGFNFTDYYIHWVRQRTEQGLEWIGRIDPENDETT
YAPKFQGKATMTADTSSNTAYLQLTSLTSEDTAVYYCARSTVLDYWGHGTTLTVSSESA
RNPTIYPLTLPRALSSDPVIIGCLIHDYFPSGTMNVTWGKSGKDITTVNFPPALASGGG
YTMSSQLTLPAVECPEGESVKCSVQHDSNAVQELDVKCSGPPPPCPPCPPSCHPSLSLQ
RPALEDLLLGSDASLTCTLNGLRNPEGAVFTWEPSTGKDAVQKKAVQNSCGCYSVSSVL
PGCAERWNSGASFKCTVTHPESDTLTGTIAKITVNTFPPQVHLLPPPSEELALNELVSL
TCLVRAFNPKEVLVRWLHGNEELSPESYLVFEPLKEPGEGATTYLVTSVLRVSAELWKQ
GDQYSCMVGHEALPMNFTQKTIDRLSGKPTNVSVSVIMSEGDGICYSSADDAKKDAAAA
DDKKADDAKKDASNSEGQIPLGGDLAPQMLRELQETNAALQDVRELLRQQVKEITFLKN
TVMECDACGMQPARTPG
> SEQ ID NO: 15: direct-comp W27 IgA H amino acid sequence (without signal sequence) 
EVQLQQSGSELVKSGASVKLSCTVSGFNFTDYYIHWVRQRTEQGLEWIGRIDPENDETT
YAPKFQGKATMTADTSSNTAYLQLTSLTSEDTAVYYCARSTVLDYWGHGTTLTVSSESA
RNPTIYPLTLPRALSSDPVIIGCLIHDYFPSGTMNVTWGKSGKDITTVNFPPALASGGG
YTMSSQLTLPAVECPEGESVKCSVQHDSNAVQELDVKCSGPPPPCPPCPPSCHPSLSLQ
RPALEDLLLGSDASLTCTLNGLRNPEGAVFTWEPSTGKDAVQKKAVQNSCGCYSVSSVL
PGCAERWNSGASFKCTVTHPESDTLTGTIAKITVNTFPPQVHLLPPPSEELALNELVSL
TCLVRAFNPKEVLVRWLHGNEELSPESYLVFEPLKEPGEGATTYLVTSVLRVSAELWKQ
GDQYSCMVGHEALPMNFTQKTIDRLSGKPTNVSVSVIMSEGDGICYNSEGQIPLGGDLA
PQMLRELQETNAALQDVRELLRQQVKEITFLKNTVMECDACGMQPARTPG
> SEQ ID NO: 16: notail-comp W27 IgA H amino acid sequence (without signal sequence)

EVQLQQSGSELVKSGASVKLSCTVSGFNFTDYYIHWVRQRTEQGLEWIGRIDPENDETT
YAPKFQGKATMTADTSSNTAYLQLTSLTSEDTAVYYCARSTVLDYWGHGTTLTVSSESA
RNPTIYPLTLPRALSSDPVIIGCLIHDYFPSGTMNVTWGKSGKDITTVNFPPALASGGG
YTMSSQLTLPAVECPEGESVKCSVQHDSNAVQELDVKCSGPPPPCPPCPPSCHPSLSLQ
RPALEDLLLGSDASLTCTLNGLRNPEGAVFTWEPSTGKDAVQKKAVQNSCGCYSVSSVL
PGCAERWNSGASFKCTVTHPESDTLTGTIAKITVNTFPPQVHLLPPPSEELALNELVSL
TCLVRAFNPKEVLVRWLHGNEELSPESYLVFEPLKEPGEGATTYLVTSVLRVSAELWKQ
GDQYSCMVGHEALPMNFTQKTIDRLSGKNSEGQIPLGGDLAPQMLRELQETNAALQDVR
ELLRQQVKEITFLKNTVMECDACGMQPARTPG

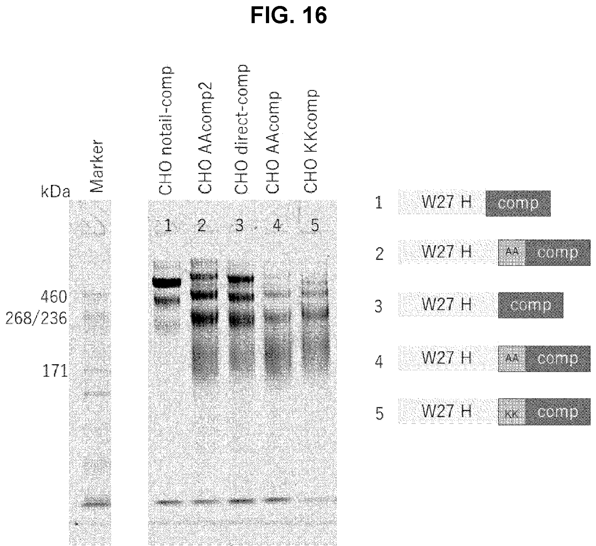

As illustrated in Fig. 16, it was confirmed that the configuration of the H chain C-terminal region of IgA and the configuration of the linker can affect the multimer formation of IgA. In particular, it was observed that, in a case where the H chain to which the COMP sequence was bound was used without using the linker, particularly in a case where the H chain to which the COMP sequence was bound by partially deleting a tail piece region of IgA was used, a ratio of IgA produced as a multimer increased.

### Example 4: Binding Properties of Multimeric Immunoglobulin Produced Using Genetically Modified Filamentous Fungus

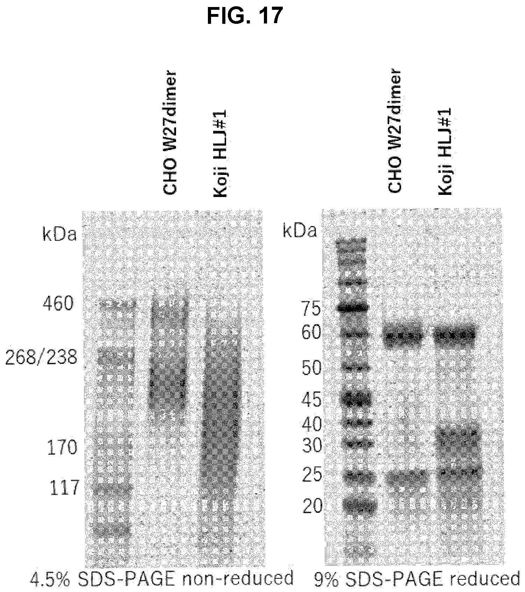

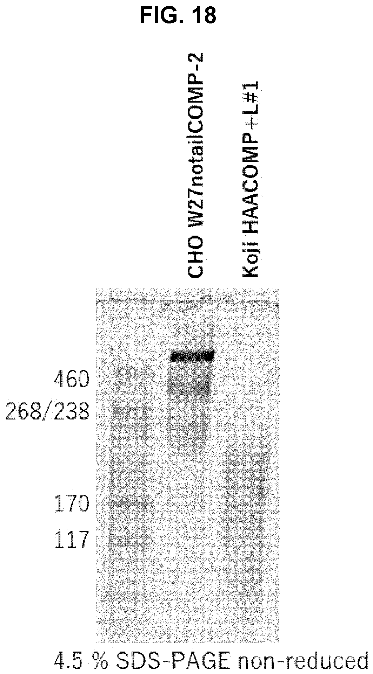

The binding force of multimerized W27 IgA secreted from a koji mold and a CHO cell to E. coli was confirmed. Note that, as described in WO 2014/142084 A, it is known that the W27 IgA antibody expressed in mammalian cells binds to E. coli. The multimerized W27 IgA secreted from a koji mold and a CHO cell was purified to test the E. coli binding ability by these multimeric IgA. The used multimeric IgA was subjected to SDS-PAGE in a reduced state (left) and a non-reduced state, and the results of CBB staining are illustrated in Figs. 17 and 18. As shown in the results of SDS-PAGE in the non-reduced state, the expression of the multimeric IgA was confirmed in both of the dimeric IgA-expression-modified koji strain (Koji HLJ#1) (Fig. 17) and the pentamer IgA-expression-modified koji strain (Koji HAA COMP+L#1) (Fig. 18). Note that the IgA antibody produced by a koji mold tended to have a band spread on SDS-PAGE in a non-reduced state.

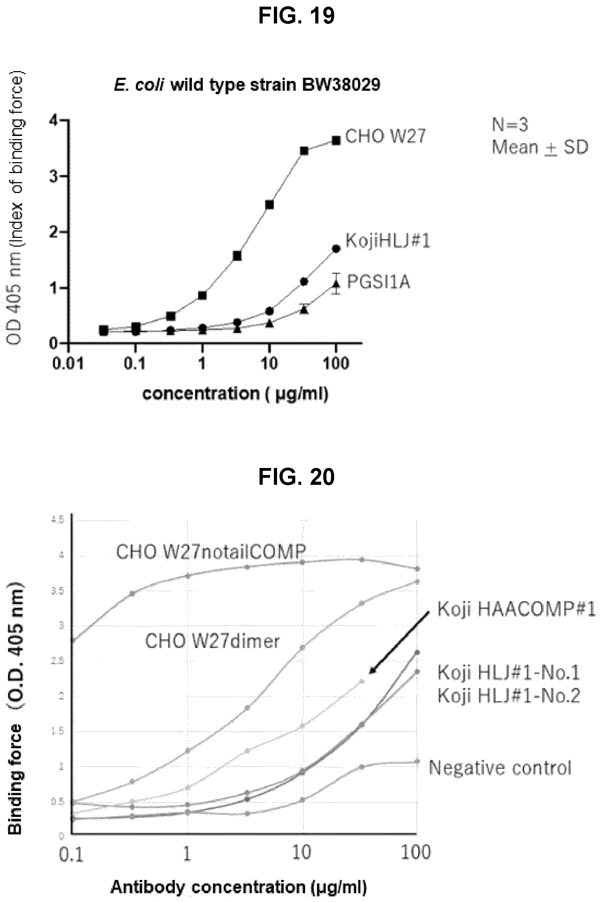

As a result of measuring the binding force to E. coli, as illustrated in Figs. 19 and 20, it was confirmed that the multimeric IgA antibody secreted from the koji mold binds to E. coli expressing SHMT protein, as in the multimeric IgA antibody secreted from CHO cell.

More specifically, Fig. 19 illustrates the test results of confirming the binding of the multimeric IgA (KojiHLJ#1) obtained from the dimeric IgA-expression-modified koji strain "H+L+J(co) #1" to the E. coli BW38029 strain.

Similarly, Fig. 20 illustrates the test results of confirming the binding of the multimeric antibody (Koji HAACOMP#1) obtained from the pentamer IgA-expression-modified koji strain "HAAComp+L(co) #1" to the E. coli BW38029 strain. For comparison, multimeric IgA (CHO W27notailCOMP) obtained from a pentamer IgA-expression-modified CHO cell to which a COMP sequence was added in a state in which a C-terminal tail piece of an IgA heavy chain was partially deficient, multimeric IgA (CHO W27dimer) expressed in a dimeric IgA-expression-modified CHO cell, multimeric IgA (Koji HLJ#1-No. 1 and Koji HLJ#1-No. 2 obtained by two separate trials were used) expressed from a dimeric IgA-expression-modified koji strain "H+L+J(co) #1", and a negative control were used (Fig. 20).

As illustrated in Fig. 20, it was confirmed that the multimeric antibody obtained from the pentamer IgA-expression-modified koji strain "HAAComp+L(co) #1" had stronger binding properties than the multimeric antibody obtained from the dimeric IgA-expression-modified koji strain "H+L+J(co) #1".

### Example 5: Physiological Activity of Multimeric Immunoglobulin Produced Using Genetically Modified Filamentous Fungus

(5-1) The growth inhibitory effect of the multimeric IgA produced by a genome-modified filamentous fungus on E. coli expressing SHMT was verified.

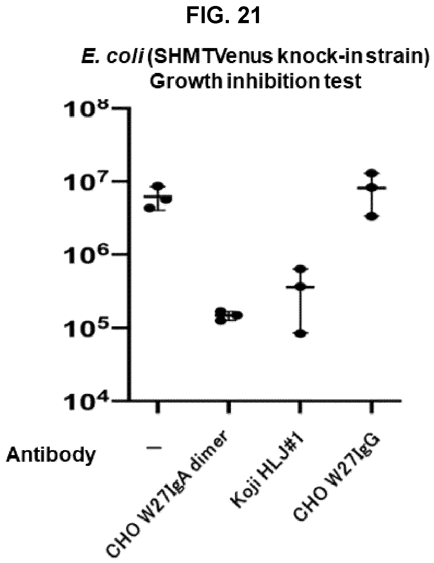

Specifically, 300 cells of E. coli (obtained by knocking in the SHMT VenusHis fusion gene to BW38029 strain) were anaerobically cultured together with a multimeric antibody (417 mg/ml) obtained from a dimeric IgA-expression-modified koji strain (KojiHLJ#1) at 37°C for 20 hours, and then seeded on a medium plate for E. coli (LB plate), and the number of colonies was measured (n=3). The results are illustrated in Fig. 21. As a comparison, a negative control containing no antibody, multimeric IgA expressed in a dimeric IgA-expression-modified CHO cell, and a W27 IgG antibody expressed in a CHO cell were used.

As in the multimeric W27IgA expressed in the dimeric IgA-expression-modified CHO cell, the multimeric W27IgA obtained from the dimeric IgA-expression-modified koji strain (KojiHLJ#1) showed a growth inhibitory effect on E. coli expressing SHMT. On the other hand, the W27 IgG antibody expressed in the CHO cell hardly exhibited such a growth inhibitory effect.

As shown by the above data, it was confirmed that the multimeric IgA antibody secreted from the multimeric IgA-expression-modified koji strain exhibited a growth inhibitory effect on E. coli, as in the multimeric IgA antibody secreted from a CHO cell.

### (5-2) The multimeric IgA produced from the genome-modified filamentous fungus was subjected to a growth inhibition test against C. difficile.

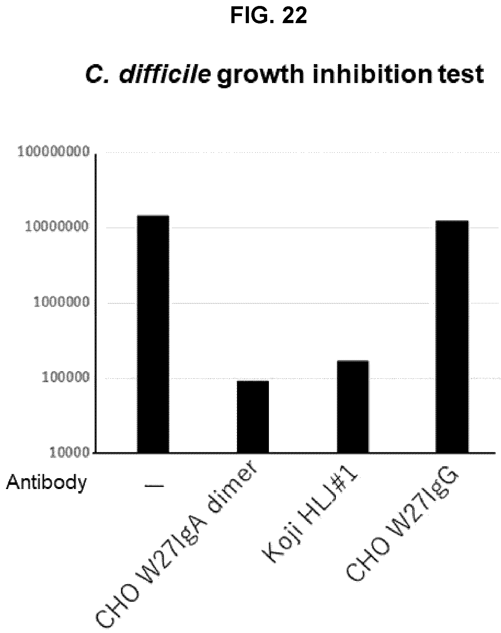

Specifically, in the same manner as in the test for E. coli, 10,000 cells of C. difficile were anaerobically cultured together with a multimeric W27 IgA antibody (417 mg/ml) obtained from a dimeric IgA-expression-modified koji strain (KojiHLJ#1) at 37°C for 20 hours, and then seeded on a medium plate (LB plate) for E. coli, and the number of colonies was measured (n=3). The results are illustrated in Fig. 22. As a comparison, a negative control containing no antibody, multimeric W27 IgA expressed in a dimeric IgA-expression-modified CHO cell, and a W27 IgG antibody expressed in a CHO cell were used. The dimeric IgA antibody (KojiHLJ#1) obtained from a koji mold showed a growth inhibitory effect on *C*. *difficile,* as in the multimeric W27 IgA expressed in the dimeric IgA-expression-modified CHO cell. On the other hand, the W27 IgG antibody expressed in the CHO cell hardly exhibited such a growth inhibitory effect.

As shown by the above data, it was confirmed that the IgA antibody secreted from a koji mold exhibited a growth inhibitory effect on C. difficile, as in the IgA antibody secreted from a CHO cell.

From the above results, it was confirmed that the multimeric IgA antibody secreted from the multimeric IgA-expression-modified koji strain has an excellent physiological activity similar to that of the multimeric IgA antibody secreted from a CHO cell.

### (References)

1.J. Yoon, J. Maruyama, K. Kitamoto (2011) Disruption of ten protease genes in the filamentous fungus Aspergillus oryzae highly improves production of heterologous proteins. Appl. Microbiol. Biotechnol., 89:747-759.
2.J. Maruyama, K. Kitamoto (2011) Targeted gene disruption in koji mold Aspergillus oryzae. Methods Mol. Biol., 765:447-456.
3.T. Katayama, H. Nakamura, Y. Zhang, A. Pascal, W. Fujii, J. Maruyama (2019) Forced recycling of an AMA1-based genome-editing plasmid allows for efficient multiple gene deletion/integration in the industrial filamentous fungus Aspergillus oryzae. Appl. Environ. Microbiol., 85:e01896-18.
4.H. H. Huynh, N. Morita, T. Sakamoto, T. Katayama, T. Miyakawa, M. Tanokura, Y. Chiba, R. Shinkura, J. Maruyama (2020) Functional production of human antibody by the filamentous fungus Aspergillus oryzae. Fungal Biol. Biotechnol., 7:7*.*

## Claims

1. A method for producing a multimeric immunoglobulin, the method comprising:
a step of preparing a filamentous fungus containing a nucleic acid fragment encoding an immunoglobulin in a genome;
a step of culturing the filamentous fungus; and
a step of separating a multimeric immunoglobulin in a culture medium from other culture medium components.

2. The method according to claim 1, wherein the filamentous fungus contains two or more copies of the nucleic acid fragment encoding the immunoglobulin in the genome.

3. The method according to claim 1 or 2, wherein the two or more copies of the nucleic acid fragment encoding the immunoglobulin are inserted into a single region of the genome of the filamentous fungus.

4. The method according to claim 1, wherein the immunoglobulin is IgA or IgM having a bacterial growth inhibitory action.

5. The method according to claim 1, wherein in the immunoglobulin, a heavy chain lacks a partial or entire region of a C-terminal tail piece.

6. A filamentous fungus comprising a nucleic acid fragment encoding an immunoglobulin in a genome, which is used in the method according to claim 1 or 2.

7. A multimeric immunoglobulin or a filamentous fungus containing a multimeric immunoglobulin, the multimeric immunoglobulin being produced by the method according to claim 1 or 2.

8. A composition comprising a multimeric immunoglobulin or a filamentous fungus containing a multimeric immunoglobulin, the multimeric immunoglobulin being produced by the method according to claim 1 or 2.

9. A method for producing a filamentous fungus containing a nucleic acid fragment encoding an immunoglobulin in a genome, the method comprising:
a step of preparing a filamentous fungus to be modified;
a step of inserting two or more copies of the nucleic acid fragment encoding the immunoglobulin in the genome into the filamentous fungus to be modified by a single-stage genome modification manipulation; and
a step of confirming that the genetically modified filamentous fungus into which the nucleic acid fragment is inserted secretes a multimeric immunoglobulin into a culture medium.

10. A genetically modified filamentous fungus comprising two or more copies of a nucleic acid fragment encoding an immunoglobulin in a genome, wherein the filamentous fungus is capable of producing a multimeric immunoglobulin.

11. The filamentous fungus according to claim 10, wherein the immunoglobulin is IgA or IgM.

12. A composition comprising the filamentous fungus according to claim 10 or 11.

13. A composition comprising a culture supernatant of the filamentous fungus according to claim 10 or 11.
